# EUROPEAN PATENT APPLICATION

(11) **EP 1 642 585 A1**
(43) Date of publication of application: **05.04.2006**
(21) Application number: 04077653.6
(22) Date of filing: 27.09.2004
(51) Int. Cl.: A61K 31/711, A61K 31/7125, A61P 19/10

(54) **Osteogenic oligonucleotides and uses thereof**

(71) Applicant: IMMUNOTECH S.A., Ciudad Autonoma de Buenos Aires (AR)
(72) Inventor: Lopez Ricardo, Augustin, Ciudad Autonoma de Buenos Aires (AR)
(74) Representative: Pistolesi, Roberto

(57) **Abstract**

Oligonucleotides having the ability to stimulate osteogenesis in vertebrate animals, including humans, are disclosed. These osteogenic oligonucleotides can be used in a wide range of clinical procedures to replace and restore osseous and periodontal defects or to facilitate the successful implantation of prosthesis and distraction procedures in bones. Methods for generation of bone in an area of an animal where skeletal tissue is deficient are provided. They consist in the local or systematic administration of a composition comprising one or more of the osteogenic oligonucleotides to the animal, in a pharmaceutically acceptable carrier. The composition is administered in an amount effective to induce osteogenesis at the bone site.

## Description

### FIELD OF THE INVENTION

This invention relates to the use of both osteogenic oligonucleotides and pharmaceutical compositions to induce bone growth in vivo. More in details it refers to oligonucleotides having about 14 to 100 nucleotides that have the ability to stimulate the osteogenesis in animals, including human.

### REFERENCES

### U.S. Patent Documents

1) United States Patent *5,409,896* April 25, 1995
2) United States Patent *5,422,340* June 6, 1995
3) United States Patent *5,604,204* February 18, 1997
4) United States Patent *5,854,207* December 29, 1998
5) United States Patent *5,885,964* March 23, 1999
6) United States Patent *5,948,428* September 7, 1999
7) United States Patent *5,972,703* October 26, 1999
8) United States Patent *6,028,207* February 22, 2000
9) United States Patent *6,355,672* March 12, 2002
10) United States Patent *6,426,222* July 30, 2002
11) United States Patent *6,531,604* March 11, 2003
12) United States Patent *6,569,204* May 27, 2003
13) United States Patent *6*,*649*,*072* November 18, 2003

### Non U.S. Patent Documents

1) WO 98/08517 March 05, 1998
2) WO 98/30234 July 16, 1998
3) WO 98/33515 August 06, 1998
4) WO 00/78351 December 28, 2000
5) WO 01/10463 February 15, 2001
6) WO 01/89521 November 29, 2001
7) WO 02/080955 October 17, 2002
8) WO 2004/030683 April 15, 2004

### Other References

1) Hughes FJ, McCulloch CA. Stimulation of the differentiation of osteogenic rat bone marrow stromal cells by osteoblast cultures. Lab Invest. 1991 May;64(5):617-22.
2) Lee SC, Shea M, Battle MA, Kozitza K, Ron E, Turek T, Schaub RG, Hayes WC.
   Healing of large segmental defects in rat femurs is aided by RhBMP-2 in PLGA matrix. J Biomed Mater Res. 1994 Oct;28(10):1149-56.
3) Asahina I, Sampath TK, Hauschka PV. Human osteogenic protein-1 induces chondroblastic, osteoblastic, and/or adipocytic differentiation of clonal murine target cells. Exp Cell Res. 1996 Jan 10;222(1):38-47.
4) Maliakal JC, Asahina I, Hauschka PV, Sampath TK. Osteogenic protein-1 (BMP-7) inhibits cell proliferation and stimulates the expression of markers characteristic of osteoblast phenotype in rat osteosarcoma (17/2.8) cells. Growth Factors. 1994;11(3):227-34.
5) Asahina I, Sampath TK, Nishimura I, Hauschka PV. Human osteogenic protein-1 induces both chondroblastic and osteoblastic differentiation of osteoprogenitor cells derived from newborn rat calvaria. J Cell Biol. 1993 Nov;123(4):921-33.
6) Akiyama H, Fukumoto A, Shigeno C, Ito H, Mukai S, Hoshino T, Makino H, Nakamura T. TAK-778, a novel synthetic 3-benzothiepin derivative, promotes chondrogenesis in vitro and in vivo. Biochem Biophys Res Commun. 1999 Jul 22;261(1):131-8.
7) Notoya K, Nagai H, Oda T, Gotoh M, Hoshino T, Muranishi H, Taketomi S, Sohda T, Makino H. Enhancement of osteogenesis in vitro and in vivo by a novel osteoblast differentiation promoting compound, TAK-778. J Pharmacol Exp Ther. 1999 Sep;290(3):1054-64.
8) Oda T, Notoya K, Gotoh M, Taketomi S, Fujisawa Y, Makino H, Sohda T. Synthesis of novel 2-benzothiopyran and 3-benzothiepin derivatives and their stimulatory effect on bone formation. J Med Chem. 1999 Feb 25;42(4):751-60.
9) Sigurdsson TJ, Nygaard L, Tatakis DN, Fu E, Turek TJ, Jin L, Wozney JM, Wikesjo UM. Periodontal repair in dogs: evaluation of rhBMP-2 carriers. Int J Periodontics Restorative Dent. 1996 Dec;16(6):524-37.
10) Hanada K, Dennis JE, Caplan Al. Stimulatory effects of basic fibroblast growth factor and bone morphogenetic protein-2 on osteogenic differentiation of rat bone marrow-derived mesenchymal stem cells. J Bone Miner Res. 1997 Oct;12(10):1606-14.
11) King GN, King N, Cruchley AT, Wozney JM, Hughes FJ. Recombinant human bone morphogenetic protein-2 promotes wound healing in rat periodontal fenestration defects. J Dent Res. 1997 Aug;76(8):1460-70.
12) Kimura M, Zhao M, Zellin G, Linde A. Bone-inductive efficacy of recombinant human bone morphogenetic protein-2 expressed in Escherichia coli: an experimental study in rat mandibular defects. Scand J Plast Reconstr Surg Hand Surg. 2000 Dec;34(4):289-99.
13) Zellin G, Linde A. Effects of recombinant human fibroblast growth factor-2 on osteogenic cell populations during orthopic osteogenesis in vivo. Bone. 2000 Feb;26(2):161-8.
14) Zellin G, Linde A. Treatment of segmental defects in long bones using osteopromotive membranes and recombinant human bone morphogenetic protein-2. An experimental study in rabbits. Scand J Plast Reconstr Surg Hand Surg. 1997 Jun;31(2):97-104.
15) Zellin G, Linde A. Importance of delivery systems for growth-stimulatory factors in combination with osteopromotive membranes. An experimental study using rhBMP-2 in rat mandibular defects. J Biomed Mater Res. 1997 May;35(2):181-90.
16) Wikesjo UM, Guglielmoni P, Promsudthi A, Cho KS, Trombelli L, Selvig KA, Jin L, Wozney JM. Periodontal repair in dogs: effect of rhBMP-2 concentration on regeneration of alveolar bone and periodontal attachment. J Clin Periodontol. 1999 Jun;26(6):392-400.
17) Barboza E, Caula A, Machado F. Potential of recombinant human bone morphogenetic protein-2 in bone regeneration.
   Implant Dent. 1999;8(4):360-7. Review.
18) Murata M, Huang BZ, Shibata T, Imai S, Nagai N, Arisue M. Bone augmentation by recombinant human BMP-2 and collagen on adult rat parietal bone. Int J Oral Maxillofac Surg. 1999 Jun;28(3):232-7.
19) Cochran DL, Schenk R, Buser D, Wozney JM, Jones AA. Recombinant human bone morphogenetic protein-2 stimulation of bone formation around endosseous dental implants. J Periodontol. 1999 Feb;70(2):139-50.
20) Isobe M, Yamazaki Y, Mori M, Ishihara K, Nakabayashi N, Amagasa T. The role of recombinant human bone morphogenetic protein-2 in PLGA capsules at an extraskeletal site of the rat. J Biomed Mater Res. 1999 Apr;45(1):36-41.
21) Mori M, Isobe M, Yamazaki Y, Ishihara K, Nakabayashi N. Restoration of segmental bone defects in rabbit radius by biodegradable capsules containing recombinant human bone morphogenetic protein-2. J Biomed Mater Res. 2000 May;50(2):191-8.
22) Hoshino T, Saito K, Muranishi H, Sohda T, Ogawa Y. Sustained-release microcapsules of a bone formation stimulant, TAK-778, for local injection into a fracture site. J Pharm Sci. 2001 Dec;90(12):2121-30.
23) Hoshino T, Muranishi H, Saito K, Notoya K, Makino H, Nagai H, Sohda T, Ogawa Y. Enhancement of fracture repair in rats with streptozotocin-induced diabetes by a single injection of biodegradable microcapsules containing a bone formation stimulant, TAK-778. J Biomed Mater Res. 2000 Sep 5;51(3):299-306.
24) Kato H, Nishiguchi S, Furukawa T, Neo M, Kawanabe K, Saito K, Nakamura T. Bone bonding in sintered hydroxyapatite combined with a new synthesized agent, TAK-778. J Biomed Mater Res. 2001 Mar 15;54(4):619-29.
25) Yudell RM, Block MS. Bone gap healing in the dog using recombinant human bone morphogenetic protein-2. J Oral Maxillofac Surg. 2000 Jul;58(7):761-6.
26) Tatakis DN, Wikesjo UM, Razi SS, Sigurdsson TJ, Lee MB, Nguyen T, Ongpipattanakul B, Hardwick R. Periodontal repair in dogs: effect of transforming growth factor-beta 1 on alveolar bone and cementum regeneration. J Clin Periodontol. 2000 Sep;27(9):698-704.
27) Danesh-Meyer MJ. Tissue engineering in periodontics and implantology using rhBMP-2. Ann R Australas Coll Dent Surg. 2000 Oct;15:144-9.
28) Oldham JB, Lu L, Zhu X, Porter BD, Hefferan TE, Larson DR, Currier BL, Mikos AG, Yaszemski MJ. Biological activity of rhBMP-2 released from PLGA microspheres. J Biomech Eng. 2000 Jun;122(3):289-92.
29) Marukawa E, Asahina I, Oda M, Seto I, Alam MI, Enomoto S. Bone regeneration using recombinant human bone morphogenetic protein-2 (rhBMP-2) in alveolar defects of primate mandibles. Br J Oral Maxillofac Surg. 2001 Dec;39(6):452-9.
30) Seto I, Asahina 1, Oda M, Enomoto S. Reconstruction of the primate mandible with a combination graft of recombinant
   human bone morphogenetic protein-2 and bone marrow. J Oral Maxillofac Surg. 2001 Jan;59(1):53-61
31) Sykaras N, Triplett RG, Nunn ME, Iacopino AM, Opperman LA. Effect of recombinant human bone morphogenetic protein-2 on bone regeneration and osseointegration of dental implants. Clin Oral Implants Res. 2001 Aug;12(4):339-49.
32) Wikesjo UM, Sorensen RG, Wozney JM. Augmentation of alveolar bone and dental implant osseointegration: clinical
   implications of studies with rhBMP-2. J Bone Joint Surg Am. 2001 ;83-A Suppl 1(Pt2):S136-45.
33) Majumdar MK, Wang E, Morris EA. BMP-2 and BMP-9 promotes chondrogenic differentiation of human multipotential mesenchymal cells and overcomes the inhibitory effect of 1L-1. J Cell Physiol. 2001 Dec;189(3):275-84.
34) Takayama S, Murakami S, Shimabukuro Y, Kitamura M, Okada H. Periodontal regeneration by FGF-2 (bFGF) in primate models.
   J Dent Res. 2001 Dec;80(12):2075-9.
35) Ripamonti U, Crooks J, Petit JC, Rueger DC. Periodontal tissue regeneration by combined applications of recombinant human
   osteogenic protein-1 and bone morphogenetic protein-2. A pilot study in Chacma baboons (Papio ursinus). Eur J Oral Sci. 2001 Aug;109(4):241-8.
36) Hoshino T, Saito K, Muranishi H, Sohda T, Ogawa Y. Sustained-release microcapsules of a bone formation stimulant, TAK-778, forlocal injection into a fracture site. J Pharm Sci. 2001 Dec;90(12):2121-30.
37) Kato H, Neo M, Tamura J, Nakamura T. Bone bonding in bioactive glass ceramics combined with a new synthesized agent
   TAK-778. J Biomed Mater Res. 2001 Nov;57(2):291-9.
38) Kato H, Nishiguchi S, Furukawa T, Neo M, Kawanabe K, Saito K, Nakamura T. Bone bonding in sintered hydroxyapatite combined with a new synthesized agent, TAK-778. J Biomed Mater Res. 2001 Mar 15;54(4):619-29.
39) Gotoh M, Notoya K, Ienaga Y, Kawase M, Makino H. Enhancement of osteogenesis in vitro by a novel osteoblast
   differentiation-promoting compound, TAK-778, partly through the expression of Msx2. Eur J Pharmacol. 2002 Sep 6;451(1):19-25.
40) Anusaksathien O, Giannobile WV. Growth factor delivery to re-engineer periodontal tissues.Curr Pharm Biotechnol. 2002 Jun;3(2):129-39.
41) Lieberman JR, Daluiski A, Einhorn TA. The role of growth factors in the repair of bone. Biology and clinical applications. J Bone Joint Surg Am. 2002 Jun;84-A(6):1032-44.
42) Saltzman WM, Olbricht WL. Building drug delivery into tissue engineering. Nat Rev Drug Discov. 2002 Mar;1(3):177-86.
43) Li G, Bouxsein ML, Luppen C, Li XJ, Wood M, Seeherman HJ, Wozney JM, Simpson H. Bone consolidation is enhanced by rhBMP-2 in a rabbit model of distraction osteogenesis. J Orthop Res. 2002 Jul;20(4):779-88.
44) Weber FE, Eyrich G, Gratz KW, Maly FE, Sailer HF. Slow and continuous application of human recombinant bone morphogenetic protein via biodegradable poly(lactide-co-glycolide) foamspheres. Int J Oral Maxillofac Surg. 2002 Feb;31(1):60-5.
45) King GN, Cochran DL. Factors that modulate the effects of bone morphogenetic protein-induced
   periodontal regeneration: a critical review. J Periodontol. 2002 Aug;73(8):925-36.
46) Schilephake H. Bone growth factors in maxillofacial skeletal reconstruction. Int J Oral Maxillofac Surg. 2002 Oct;31 (5):469-84.
47) Ikeuchi M, Dohi Y, Horiuchi K, Ohgushi H, Noshi T, Yoshikawa T, Yamamoto K,
   Sugimura M. Recombinant human bone morphogenetic protein-2 promotes osteogenesis within atelopeptide type I collagen solution by combination with rat cultured marrowcells. J Biomed Mater Res. 2002 Apr;60(1):61-9.
48) Marukawa E, Asahina I, Oda M, Seto I, Alam M, Enomoto S. Functional reconstruction of the non-human primate mandible using recombinant human bone morphogenetic protein-2. Int J Oral Maxillofac Surg. 2002 Jun;31(3):287-95.
49) Selvig KA, Sorensen RG, Wozney JM, Wikesjo UM. Bone repair following recombinant human bone morphogenetic protein-2 stimulated periodontal regeneration. J Periodontol. 2002 Sep;73(9):1020-9.
50) Rosa AL, Beloti MM. TAK-778 enhances osteoblast differentiation of human bone marrow cells. J Cell Biochem. 2003 Aug 15;89(6):1148-53.
51) Cook SD, Barrack RL, Patron LP, Salkeld SL. Osteoinductive agents in reconstructive hip surgery: a look forward.
   Clin Orthop. 2003 Dec;(417):195-202.
52) Puleo D. Biotherapeutics in orthopaedic medicine: accelerating the healing process? BioDrugs. 2003;17(5):301-14.
53) Tabata Y. Tissue regeneration based on growth factor release. Tissue Eng. 2003;9 Suppl 1:S5-15.
54) Wikesjo UM, Xiropaidis AV, Thomson RC, Cook AD, Selvig KA, Hardwick WR. Periodontal repair in dogs: rhBMP-2 significantly enhances bone formation under provisions for guided tissue regeneration. J Clin Periodontol. 2003 Aug;30(8):705-14.
55) Matin K, Senpuku H, Hanada N, Ozawa H, Ejiri S. Bone regeneration by recombinant human bone morphogenetic protein-2 around
   immediate implants: a pilot study in rats. Int J Oral Maxillofac Implants. 2003 Mar-Apr;18(2):211-7.
56) Nakashima M, Reddi AH. The application of bone morphogenetic proteins to dental tissue engineering. Nat Biotechnol. 2003 Sep;21(9):1025-32.
57) Camelo M, Nevins ML, Schenk RK, Lynch SE, Nevins M. Periodontal regeneration in human Class II furcations using purified
   recombinant human platelet-derived growth factor-BB (rhPDGF-BB) with bone allograft. Int J Periodontics Restorative Dent. 2003 Jun;23(3):213-25.
58) Sheehan JP, Sheehan JM, Seeherman H, Quigg M, Helm GA. The safety and utility of recombinant human bone morphogenetic protein-2 for cranial procedures in a nonhuman primate model. J Neurosurg. 2003 Jan;98(1):125-30.
59) Murakami S, Takayama S, Kitamura M, Shimabukuro Y, Yanagi K, Ikezawa K, Saho T, Nozaki T, Okada H. Recombinant human basic fibroblast growth factor (bFGF) stimulates periodontal regeneration in class II furcation defects created in beagle dogs.
   J Periodontal Res. 2003 Feb;38(1):97-103.
60) Rasubala L, Yoshikawa H, Nagata K, lijima T, Ohishi M. Platelet-derived growth factor and bone morphogenetic protein in the healing of mandibular fractures in rats. Br J Oral Maxillofac Surg. 2003 Jun;41 (3):173-8.
61) Clokie CM, Bell RC. Recombinant human transforming growth factor beta-1 and its effects on osseointegration. J Craniofac Surg. 2003 May;14(3):268-77.
62) Lee YM, Nam SH, Seol YJ, Kim TI, Lee SJ, Ku Y, Rhyu IC, Chung CP, Han SB, Choi SM. Enhanced bone augmentation by controlled release of recombinant human bone morphogenetic protein-2 from bioabsorbable membranes. J Periodontol. 2003 Jun;74(6):865-72.
63) Sykaras N, Opperman LA. Bone morphogenetic proteins (BMPs): how do they function and what can they offer the clinician? J Oral Sci. 2003 Jun;45(2):57-73.
64) Sykaras N, Opperman LA. Bone morphogenetic proteins (BMPs): how do they function and what can they offer the clinician? J Oral Sci. 2003 Jun;45(2):57-73.
65) Schmidmaier G, Wildemann B, Gabelein T, Heeger J, Kandziora F, Haas NP, Raschke M. Synergistic effect of IGF-I and TGF-betal on fracture healing in rats: single versus combined application of IGF-I and TGF-betal. Acta Orthop Scand. 2003 Oct;74(5):604-1 0.
66) Cheng H, Jiang W, Phillips FM, Haydon RC, Peng Y, Zhou L, Luu HH, An N, Breyer B, Vanichakarn P, Szatkowski JP, Park JY, He TC. Osteogenic activity of the fourteen types of human bone morphogenetic proteins (BMPs). J Bone Joint Surg Am. 2003 Aug;85-A(8):1544-52.
67) van den Dolder J, de Ruijter AJ, Spauwen PH, Jansen JA. Observations on the effect of BMP-2 on rat bone marrow cells cultured on titanium substrates of different roughness. Biomaterials. 2003 May;24(11):1853-60.
68) Matin K, Senpuku H, Hanada N, Ozawa H, Ejiri S. Bone regeneration by recombinant human bone morphogenetic protein-2 around
   immediate implants: a pilot study in rats. Int J Oral Maxillofac Implants. 2003 Mar-Apr;18(2):211-7.
69) Wikesjo UM, Qahash M, Thomson RC, Cook AD, Rohrer MD, Wozney JM, Hardwick WR. Space-providing expanded polytetrafluoroethylene devices define alveolar augmentation at dental implants induced by recombinant human bone morphogenetic protein 2 in an absorbable collagen sponge carrier. Clin Implant Dent Relat Res. 2003;5(2):112-23.
70) Wikesjo UM, Qahash M, Thomson RC, Cook AD, Rohrer MD, Wozney JM, Hardwick WR. rhBMP-2 significantly enhances guided bone regeneration. Clin Oral Implants Res. 2004 Apr;15(2):194-204.
71) Benghuzzi H, Tucci M, Tsao A, Russell G, England B, Ragab A. Stimulation of osteogenesis by means of sustained delivery of various natural androgenic hormones. Biomed Sci Instrum. 2004;40:99-104.
72) Rosa AL, Beloti MM. TAK-778 enhances osteoblast differentiation of human bone marrow cells via an
   estrogen-receptor-dependent pathway. J Cell Biochem. 2004 Mar 1;91(4):749-55.
73) Roldan JC, Jepsen S, Miller J, Freitag S, Rueger DC, Acil Y, Terheyden H. Bone formation in the presence of platelet-rich plasma vs. bone morphogenetic protein-7. Bone. 2004 Jan;34(1):80-90.
74) Ashinoff RL, Cetrulo CL Jr, Galiano RD, Dobryansky M, Bhatt KA, Ceradini DJ, Michaels J 5th, McCarthy JG, Gurtner GC.
   Bone morphogenic protein-2 gene therapy for mandibular distraction osteogenesis. Ann Plast Surg. 2004 Jun;52(6):585-90.
75) Osyczka AM, Diefenderfer DL, Bhargave G, Leboy PS. Different effects of BMP-2 on marrow stromal cells from human and rat bone. Cells Tissues Organs. 2004;176(1-3):109-19.

### BACKGROUND OF THE INVENTION

Bone is a complex, highly organized, connective tissue that is continuously remodelled during the life of an adult by cellular events that initially break it down (osteoclastic resorption) and then rebuild it (osteoblastic formation). This remodelling process occurs in discrete sites throughout the skeleton. Bone is the only organ capable of complete repair without the intervention of a fibrous scar (Hult. A. 1989. Current concepts of fracture healing. Clin. Orthop. Relat. Res. 249:265-384.). However, there are clinical situations that require enhancement of the healing to ensure the rapid restoration of bone function, such as orthopaedic and maxillofacial surgery. On the other hand, some events, including aging, poor blood supply, and diabetes, may lead to prevent fracture healing (J.A. Buckwalter, T.A. Einhorn, M.E. Bolander and R.L. Cruess , Healing of the musculoskeletal tissues. In: C.A. Rockwood, D.P. Green, R.W. Bucholz and J.D. Heckman, Editors, Fracture in Adults, Lippincott-Raven, New York (1996), pp. 261-304.). The restoration of an osteoporotic fracture is also delayed since the mechanical strength of the fracture site is decreased due to an insufficient amount of callus and calcification (L.J. Melton, III , Epidemiology of fractures. In: B.L. Riggs and L.J. Melton, III, Editors, Osteoporosis: Etiology, Diagnosis, and Management, Lippincott-Raven, Philadelphia (1995), pp. 225-247. ; W.R. Walsh, P. Sherman, C.R. Howlett, D.H. Sonnabend and M.G. Ehrlich, Fracture healing in a rat osteopenia model. Clin. Orthop. Relat. Res. 342 (1997), pp. 213-227.). Thus, the development of agents to enhance osteogenesis would be a significant pharmacological advancement in terms of accelerating both fracture healing and the surgical procedures involving bone repair and of preventing fractures in patients suffering osteogenic dysfunctions.

A number of growth factors and cytokines are present in high levels at fracture sites and many of these proteins play important roles in promoting bone repair (M.E. Bolander, Regulation of fracture repairs by growth factors. Proc. Soc. Exp. Biol. Med. 200 (1992), pp. 165-170.). Drug therapies using various growth factors such as bone morphogenetic proteins (BMPs), transforming growth factor β (TGF- β), insulin -like growth factors (IGFs), fibroblast growth factors (FGFs), and platelet-derived growth factor (PDGF) may be useful since local application of these molecules has been shown to induce bone regeneration in animal models (Lind M (1996) Growth factors: Possible new clinical tools. Acta Orthop. Scand. 67: 407-417).

These findings indicate that the clinical use of these growth factors may become possible new therapies for enhancing fracture healing. However, the safety and cost-effectiveness of these growth factors must be considered. Therefore, there has been substantial interest in developing chemical compounds that safely promote bone formation and facilitate fracture repair. One example of such a compound is the TAK-778, a derivative of the ipriflavone (7-isopropoxy-isoflavone) which has osteogenic activity "in vitro" and "in vivo" (: Notoya K, Nagai H, Oda T, Gotoh M, Hoshino T, Muranishi H, Taketomi S, Sohda T, Makino H (1999). Enhancement of osteogenesis in vitro and in vivo by a novel osteoblast differentiation promoting compound, TAK-778.J Pharmacol Exp Ther. 290:1054-64). Utility of this compound for human use must be proved in clinical trials.

We now disclose that oligonucleotides having about 14 to 100 nucleotides are compounds with potent osteogenic activity.

It is an object of the present invention to provide one or more of the osteogenic oligonucleotides of this invention to an animal with a skeletal (bony) tissue deficiency to produce mature, morphologically normal bone where it is needed.

This object will become apparent to those skilled in the art.

### SUMMARY OF THE INVENTION

The above object is achieved by providing a method for bone generation at a site of an animal where skeletal tissue is deficient and which consists in the administration of an effective amount of a composition comprising one or more of the osteogenic oligonucleotides of this invention to the animal, locally at the site or systemically as needed in each case, in a pharmaceutically acceptable carrier, the composition being administered in an amount effective to induce bone growth at the site.

This aspect of the invention enables the generation of normal mature bone in the skeleton in general or locally as required. Pre-clinical results using as example some of the osteogenic ODNs of this invention described below show new bone formation in bone defects in rats, and new bone formation in bone defects in primates.

### BRIEF DESCRIPTION OF THE DRAWINGS

Fig.1 shows an X-ray radiographic analysis of the tibias of a rat, at osteotomy sites, 7, 21 and 35 days after operation, both in the placebo (A, B and C) and in IMT504-treated (D, E and F) bone.
Fig. 2 shows photographs of the tibias of a rat, at osteotomy sites, 35 days after operation both in the placebo (A) and in IMT504-treated (B) bone.
Fig. 3 shows microphotographs of the tibias of a rat, at osteotomy sites, 35 days after operation both in the placebo (A) and in IMT504-treated (B) bone.

### DESCRIPTION OF THE PREFERRED EMBODIMENTS

"Inducing bone growth" means promoting the formation of morphologically normal, mature bone only at a site where there is a bone deficiency that needs to be replaced. Mature bone is bone of any type, whether cortical or trabecular, that is mineralized as opposed to immature or cartilaginous bone as would be formed in a neonatal model. Morphologically normal bone is bone that is histologically detected as normal (i.e., consisting of endochondral or membranous type lamellar bone and including marrow spaces with osteoblasts and osteoclasts). This is in contrast, for example, to callous formation with a fibrotic matrix as seen in the first stage of fracture healing. Thus, the bone induction herein is contemplated not only as acceleration of bone regeneration, as in a fracture, but also as stimulation of the formation of bone that is returned to its normal morphological state.

"Skeletal tissue deficiency" refers to a deficiency in bone at any site, originated as a result of either surgical intervention or fracture, and where bone it is desired to restore the bone.

By "osteogenesis" is meant the process by which bone develops.

By "osteogenic" cells is meant cells able to proliferate and to differentiate into osteoblasts and osteocytes.

By "osteoblasts" is meant mature bone cell concerned with synthesis and secretion of bone extracellular organic constituents.

By "osteocytes" is meant cells that are essentially osteoblasts surrounded by the products they secrete.

By "animal" is meant any animal having a vertebrate structure, preferably a mammal, and most preferably a human.

A "subject" refers to an animal of the order primate, including humans.

As used herein, the term "oligonucleotide" or "oligo" shall mean multiple nucleotides (i.e. molecules comprising a sugar, e.g. ribose or deoxyribose, linked to a phosphate group and to an exchangeable organic base, which is either a substituted pyrimidine (e.g. cytosine (C), thymine (T) or uracil (U)) or a substituted purine (e.g. adenine (A) or guanine (G)). The term "oligonucleotide" as used herein refers to both oligoribonucleotides (ORNs) and oligodeoxyribonucleotides (ODNs). The term "oligonucleotide" shall also include oligonucleosides (i.e. an oligonucleotide minus the phosphate) and any other organic base containing polymer. Oligonucleotides can be obtained from existing nucleic acid sources (e.g. genomic or cDNA), but are preferably synthetic (e.g. produced by oligonucleotide synthesis).

An "oligonucleotide" refers to multiple nucleotides linked by phosphodiester bonds.

An "immunostimulatory oligonucleotide" refers to an oligonucleotide which stimulates (i.e. has a mitogenic effect on, induces, increases or decreases cytokine expression by) a cell of the immune system (i.e. a lymphocyte or a macrophage) in a statistically significant manner.

A "CpG" refers to a cytosine-guanine dinucleotide.

A "CpG oligonucleotide" refers to an oligonucleotide which stimulates a cell of the immune system, and whose immunostimulatory activity critically depends on the presence of at least one CpG in its sequence.

A "non-CpG oligonucleotide" refers to an oligonucleotide that stimulates a cell of the immune system, and whose immunostimulatory activity does not critically depend on the presence of a CpG in its sequence.

### Modes for Carrying Out the Invention

The invention is carried out in one aspect by mixing one or more of the osteogenic oligonucleotides of this invention with a suitable pharmaceutical carrier and by administering the resulting composition locally or systemically as required to an animal in order to induce formation of normal, adult bone in bone lesions. Osteogenic cells and their precursor cells should be present at the lesion site or sites. If the lesion site or sites does not naturally have a source of osteogenic cells present, the pharmaceutical composition may also contain an osteogenic cell source, in an amount sufficient to induce bone growth.

Examples of indications where promotion of bone repair at a skeletal site or sites is important include: periodontal disease where root socket healing is impaired (tooth socket sites), non-union fractures (including primary treatment of high risk fractures and adjunctive treatment with bone grafting or bone substitutes for established non-union fractures), large bony defects caused by trauma or surgery [e.g., partial mandibular resection for cancer, large cranial defects, spinal (vertebral) fusions, correction of severe scoliosis by surgical alignment held in place with a Harrington bar (to shorten the six-month period normally required for a body cast), and spinal fractures with open reduction (to decrease significantly the period of immobilization)], and rapid stabilization and enhanced fixation of artificial prostheses and spacer bars, oral joints, and bone replacements.

Examples of the latter include plastic and reconstructive surgery, fixation of permanent dentures into mandible, enhanced fixation of accepted Joint prosthesis, e.g., hips, knees, and shoulders (leading to the acceptance of prostheses that are unacceptable due to rapid loosening and instability), and limb salvage procedures, usually associated with malignancy (the bone shaft may be removed but the articular surfaces are left in place and connected by a space bar; rapid and enhanced fixation is required for success). If the site constitutes a periodontal site, i.e., one that involves the teeth, gums, and dental sockets, the osteogenic oligonucleotides of this invention could be administered in conjunction with an exogenously added source of osteogenic cells.

In one preferred embodiment, the osteogenic oligonucleotides of this invention are administered by coating a device with the composition containing one or more of the oligonucleotides of this invention and by implanting the device into the animal at the site of the deficiency. The composition may also contain an osteogenic cell source when the site is deficient in such cells. The device may consist in any device suitable for implantation, including a molded implant, plug, prosthetic device, capsule, titanium alloy, sponge, or ceramic block. Examples of suitable delivery vehicles useful as devices are those disclosed by Nade et al., Clin. Orthop. Rel. Res., 181: 255-263 (1982); Uchida et al., J. Biomed. Mat. Res., 21: 1-10 (1987); Friedenstein et al., Exp. Hematol., 10: 217-227 (1982); Deporter et al., Calcif. Tissue Int., 42: 321-325 (1988); McDavid et al., J. Dent. Res., 58: 478-483 (1979); Ohgushi et al., J. Orthopaedic Res., 7: 568-578 (1989), Aprahamian et al., J. Biomed. Mat. Res., 21: 965-977 (1986) and Emmanual et al., Stain. Tech., 62: 401-409 (1987).

For bone defects involving gaps, such as a dry socket or a non-union fracture, a plug may be used to fill the gap. The plug may be composed of, for example, hydroxyapatite or collagen on which the composition containing one or more of the oligonucleotides of this invention adsorbed. For larger bone defects resulting from, e.g., trauma or skeletal reconstruction around an ulcer or hip prosthesis, the device is preferably a made-to-fit ceramic block. More preferably, the ceramic block comprises 0-100% hydroxyapatite and the remaining 100-0% tricalcium phosphate, by weight, most preferably 60% hydroxyapatite and 40% tricalcium phosphate.

In a specific embodiment for a jaw implant, a calcium carbonate moldable material or Interpore.TM. molding device is molded to fit the jaw, using a 3-dimensional x-ray of the jaw before surgery. The molded material is impregnated with the composition containing one or more of the oligonucleotides of this invention. Then, dispensed bone marrow from another site of the animal (e.g., from the hip) is infiltrated into the mold, and the mold is placed into the jaw for final implantation.

Preferably, the device is treated with the composition containing one or more of the oligonucleotides of this invention (e.g. solution or gel) for a sufficient period of time to allow adsorption. Both the concentration of the oligonucleotides of this invention in the solution or gel and the time of exposure depend on a number of factors, including the volume of the defect and the nature of the site to which it is applied, and should be adjusted accordingly. As the size of the defect increases, or when the site is other than a bone site, the concentration of the oligonucleotides and the time of pre-soaking should be increased. The treatment should preferably be for at least about 0.5 hour, depending on the factors mentioned above (more preferably at least about 1 hour, and most preferably 1-2 hours), before implantation. Also depending on the above considerations, the concentration of oligonucleotides in the composition should preferably be of at least about 0.1 mg/ml (more preferably of at least about 0.5-10 and up to 100 mg/ml). The treatment may consist of any mode by which the composition is applied to the device to deliver effectively the osteogenic oligonucleotides of this invention and the osteogenic cell source if necessary. Such treatment includes, for example, adsorption or impregnation, depending in part on the nature of the indication.

The compositions containing the osteogenic oligonucleotides of this invention to be used in the therapy will be dosed in a fashion consistent with good medical practice taking into account the nature of the skeletal tissue deficiency to be treated, the species of the host, the medical condition of the individual patient, the presence of any other drug in the composition, the site of delivery of the agent, the method of administration, the scheduling of administration, and other factors known to practitioners. Because of differences in host response, significant site-to-site and patient-to-patient variability exists. For purposes herein, the "therapeutically effective amount" of the osteogenic oligonucleotides of this invention is an amount that is effective to induce bone growth, as defined above, at the site of skeletal tissue deficiency.

As a general proposition, the osteogenic oligonucleotides of this invention are formulated and delivered to the target site at a dosage capable of establishing an oligonucleotide level equal or greater than about 0.1 mg/ml at the site. Typically, the oligonucleotide concentrations range from about 0.1 mg/ml to 12 mg/ml, preferably from about I to 4 mg/ml. These intra-tissue concentrations are maintained preferably by topical application and/or sustained release.

As noted above, these suggested amounts of oligonucleotides are subject to a great deal of therapeutic discretion. The key factor in selecting an appropriate dose and scheduling is the result obtained. Clinical parameters to determine an endpoint include increase in bone formation and mass and in radiographically detectable bone height. Such measurements are well known to those clinicians and pharmacologists skilled in the art.

The oligonucleotide composition is administered either locally to the site by any suitable means, including topical and continuous release formulation, or systemically, as needed. The oligonucleotides are generally combined at ambient temperature at the appropriate pH, and at the desired degree of purity, with a physiologically acceptable carrier, i.e., a carrier that is non-toxic to the patient at the dosages and concentrations employed. The carrier may take a wide variety of forms depending on the form of preparation desired for administration.

For the preparation of a liquid composition suitable for impregnation of a device, the carrier is a suitable buffer, a low molecular weight (less than about 10 residues) polypeptide, a protein, an amino acid, a carbohydrate (including glucose or dextrans), a chelating agent such as EDTA, cellulose, or other excipients. In addition, the oligonucleotide composition is preferably sterile. Sterility is readily accomplished by sterile filtration through 0.2 micron membranes. The oligonucleotide will be ordinarily stored as an aqueous solution, although lyophilized formulations for reconstitution are acceptable.

Generally, where the bone disorder allows so, one should formulate and dose the oligonucleotide for site-specific delivery, where the oligonucleotide is formulated into a sterile sustained-release composition suitable for local application to the desired site.

For local application of the oligonucleotide composition, for example, in the case of a bone defect that is a crack, e.g., a union fracture; the carrier may be any vehicle effective for this purpose. For obtaining a gel formulation, the liquid composition is typically mixed with an effective amount of a water-soluble polysaccharide, polyethylene glycol, or synthetic polymer such as polyvinylpyrrolidone to form a gel of the proper viscosity to be applied topically. The polysaccharide is generally present in a gel formulation in the range of 1-90% by weight of the gel, more preferably 1-20%. The polysaccharide that may be used includes, for example, cellulose derivatives such as etherified cellulose derivatives, including alkyl celluloses, hydroxyalkyl celluloses, and alkylhydroxyalkyl celluloses, for example, methylcellulose, hydroxyethyl cellulose, carboxymethyl cellulose, hydroxypropyl methylcellulose, and hydroxypropyl cellulose; starch and fractionated starch, agar; alginic acid and alginates, gum arabic, pullullan, agarose, carrageenan, dextrans, dextrins, fructans, inulin, mannans, xylans, arabinans, chitosans, glycogens, glucans, and synthetic biopolymers, as well as gums such as xanthan gum, guar gum, locust bean gum, gum arabic, tragacanth gum, and karaya gum, and derivatives and mixtures thereof. The preferred gelling agent herein is one that is inert to biological systems, non-toxic, simple to prepare, not too runny or viscous, and one that will not destabilize the oligonucleotide held within it. Preferably the polysaccharide is an etherified cellulose derivative, more preferably one that is well defined, purified, and listed in USP, e.g., methylcellulose and the hydroxyalkyl cellulose derivatives, such as hydroxypropyl cellulose, hydroxyethyl cellulose, and hydroxypropyl methylcellulose. Most preferred herein is methylcellulose.

The polyethylene glycol useful for gelling is typically a mixture of low and high molecular weight polyethylene glycols to obtain the proper viscosity. For example, a mixture of a polyethylene glycol of molecular weight of 400-600 Dalton with one of molecular weight of 1,500 would be effective for this purpose when mixed in the proper ratio to obtain a paste. The term "water soluble" as applied to the polysaccharides and polyethylene glycols is meant to include colloidal solutions and dispersions. In general, the solubility of the cellulose derivatives is determined by the degree of substitution of ether groups, and the stabilizing derivatives useful herein should have a sufficient quantity of such ether groups per anhydroglucose unit in the cellulose chain to render the derivatives water soluble. A degree of ether substitution of at least 0.35 ether groups per anhydroglucose unit is generally sufficient. Additionally, the cellulose derivatives may be in the form of alkali metal salts, for example, the Li, Na, K, or Cs salts.

In a preferred embodiment, the gel contains about 2-5% by weight methylcellulose and the oligonucleotide is present in an amount of about 10-1000 µg per ml of gel. More preferably, the gel consists of about 3% methylcellulose by weight, lactic acid to pH 5.0, and 20-200 µg per ml of oligonucleotide.

For the preparation of a sustained-release formulation, the oligonucleotide is suitably incorporated into a biodegradable matrix or microcapsular particle. A suitable material for this purpose is a polylactide, although other polymers of poly (.alpha.-hydroxycarboxylic acids), such as poly-D-(-)-3-hydroxybutyric acid (EP 133,988A), can be used. Additional biodegradable polymers include poly(lactones), poly(acetals), poly(orthoesters) or poly(orthocarbonates). The oligonucleotide is also suitably mixed with a biodegadable protein carrier such as collagen, atelocollagen, or gelatin to form a carrier matrix having sustained-release properties; the resultant mixture is then dried, and the dried material is formed into an appropriate shape, as described in U.S. Pat. No. 4,774,091.

The initial consideration here must be that the carrier itself, or its degradation products, are non-toxic in the target bone site and will not further aggravate the condition. This can be determined by routine screening in animal models of the target bone disorder or, if such models were unavailable, in normal animals. For examples of sustained-release compositions, see U.S. Pat. No. 3,773,919, EP 58,481A, U.S. Pat. No. 3,887,699, EP 158,277A, Canadian Patent No. 1176565, U. Sidman et al., Biopolymers, 22:547 (1983), and R. Langer et al., Chem. Tech., 12:98 (1982).

Controlled delivery of the oligonucleotide to a site is also suitably accomplished using permeable hollow cellulose acetate fibers with the oligonucleotide placed in the site and removed 24 hours later or left for longer periods of time (U.S. Pat.

No. 4,175,326). Also, acrylic resin strips or cast films can be impregnated with the oligonucleotide and applied to the affected site. In addition, narrow dialysis tubing can be filled with a solution of the oligonucleotide and placed so as to deliver it to the appropriate site.

The composition herein may also suitably contain other osteogenetic factors such as IGF-1, TGF-beta 1, and PDGF. Such osteogenetic factors are suitably present in an amount that is effective for the intended purpose, i.e., to promote formation of bone.

The invention will be better understood by reference to the following examples. They should not, however, be construed as limiting the scope of the invention. All literature citations are incorporated by reference.

### EXAMPLE 1

### Materials and Methods

The following materials and methods were generally used throughout the examples.

### 1) Oligonucleotides

Oligonucleotides having phosphorothioate internucleotide linkages were purchased, purified by high-pressure liquid chromatography (HPLC), from Operon Technologies (Alameda, California) or Annovis (Aston, Pennsylvania) or Oligos Etc (Bethel, Maine). ODNs were suspended in depyrogenated water, assayed for LPS contamination using the Limulus test and kept at -20 °C until used. Purity was assessed by HPLC and PAGE assays. ODN preparations were used if LPS levels were undetectable.

### 2) Animal Experiments

Young (8-12 weeks old) adult male Spragüe Dawley rats weighing about 350 g were used. Animals were anesthetized by intraperitoneal (i. p.) injection of a mixture of ketamine (50 mg/kg) and xilacine (5 mg/kg). After skin overlying the hind limb was shaved and sterilized. A 1.5 cm longitudinal incision was performed in the tibia forefront zone. To generate a defect in the bone, an osteotomy was made using a low-speed dental drill attached to a round diamond saw under saline irrigation. For the osteotomy, a site free of muscular insertions was selected 15 mm below the ankle. The wound was deep enough to reach the bone marrow. A single dose (as stated in each experiment) of ODN in 3 µl of methylcellulose 1% was introduced into the defect made in the right tibia. As a control, the same volume (3 µl) of vehicle was introduced into the defect made in the left tibia. Fracture callus formation was evaluated radiographically on days 0, 21 and 28. On day 28, animals were euthanized under ether atmosphere, and the tibias removed and photographed. After this, the tibias were fixed in 10% formol solution, decalcified in 10% EDTA solution, and embedded in paraffin. A longitudinal section of each tibia was cut, stained with hematoxilin/eosine and examined under a light microscope.

### EXAMPLE 2

### Osteogenesis stimulation by oligonucleotides.

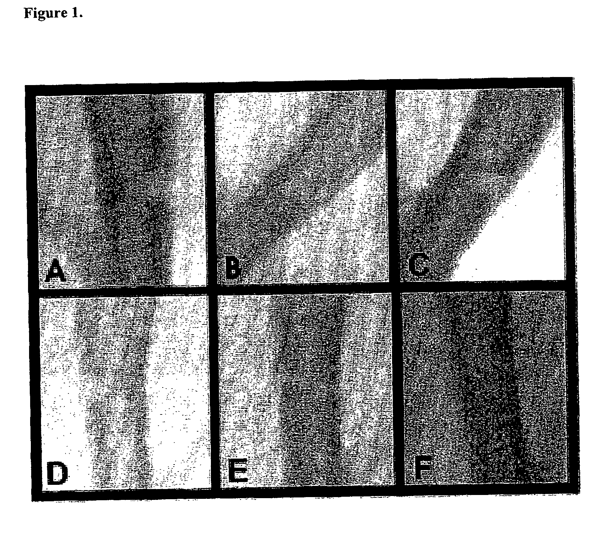

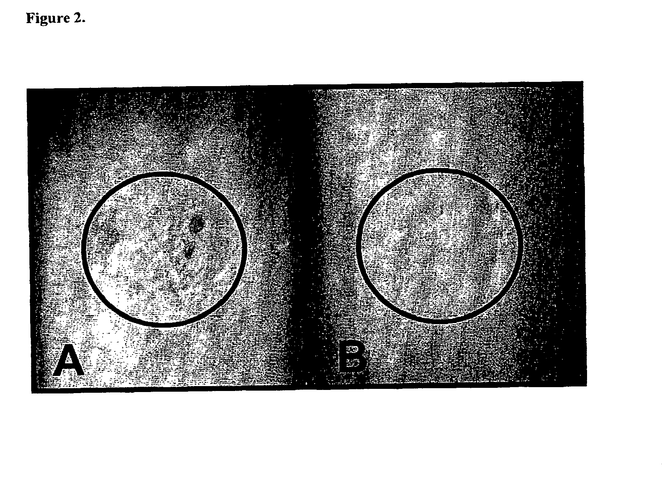

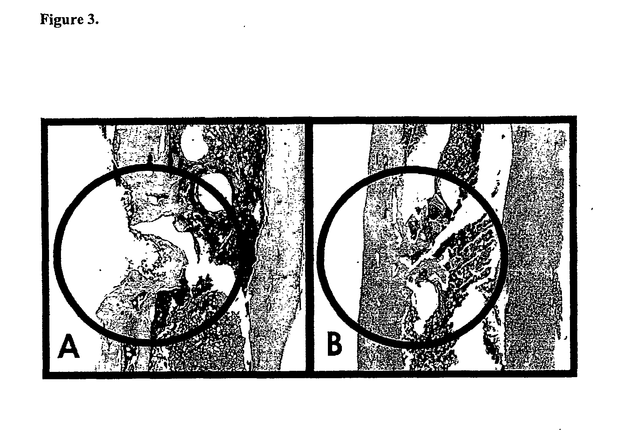

A rat femur tibial model was employed as described in Example 1. First, a total dose per defect of 60 µg of oligonucleotide was used. The oligonucleotide used in these experiments was IMT504. This oligonucleotide is 24 nucleotides long, its nucleotide sequence is 5'-TCATCATTTTGTCATTTTGTCATT-3', and all the DNA (natural) phosphodiester bonds have been replaced with phosphorothioate bonds to protect it from enzymatic degradation. As example, Fig. 1 shows radiographs corresponding to both tibias of an experimental rat. In the right tibia, were the bond defect has been filled with vehicle plus IMT504, radiodense material filling the defect can be observed as early as 3 weeks after initiation of the treatment. In this treated right tibia, the defect is no longer visible at week 5 after initiation of the treatment. On the other hand, the defect is clearly visible by week 5 in the untreated (control) left tibia. Fig.2 shows a photograph of the right (treated with IMT504) and left (untreated) tibia at the site where the experimental osteotomy was performed. As can be observed, the defect in the treated tibia appears completely filled with apparently normal bond while the defect in the control tibia is filled with spongy material corresponding to an incomplete ossification. The corresponding histological documentation can be seen in Fig. 3. In this figure the treated tibia shows well- formed bone tissue at the site where the osteotomy was performed. In contrast, in the untreated tibia the defect is still visible.

### EXAMPLE 3

### Efect of the ODN concentration in osteogenesis induction

In order to evaluate the minimal amount of IMT504 necessary to obtain rapid ossification, a rat femur tibial model was employed as described in Example 1.

Table 1 shows that filling the osteotomy with a gel containing at least 0.4 mg/ml of IMT504 is necessary to obtain rapid ossification even though some activity was observed in a concentration as low as 0.06 mg/ml.

**TABLE 1**

| | | | | | | | | |
|---|---|---|---|---|---|---|---|---|
| IMT504 concentration (mg/ml) | 0 | 0.06 | 0.4 | 0.8 | 1.5 | 3 | 6 | 12 |
| Ossification 35 days after operation | - | +/- | + | + | + | + | + | + |
| IMT504 (SEQ ID N°2) | | | | | | | | |

### EXAMPLE 4

### Induction of osteogenesis by ODNs with different composition

Several of the components of the immune-system play a role in regulation of the osteogenic proccess (Shinoda K, Sugiyama E, Taki H, Harada S, Mino T, Maruyama M, Kobayashi M. Resting T cells negatively regulate osteoclast generation from peripheral blood monocytes. Bone. 2003 Oct;33(4):711-20.; Evans DB, Bunning RA, Russell RG. The effects of recombinant human granulocyte-macrophage colony-stimulating factor (rhGM-CSF) on human osteoblast-like cells. Biochem Biophys Res Commun. 1989 Apr 28;160(2):588-95; Fujikawa Y, Sabokbar A, Neale SD, Itonaga I, Torisu T, Athanasou NA. The effect of macrophage-colony stimulating factor and other humoral factors (interleukin-1, -3, -6, and -11, tumor necrosis factor-alpha, and granulocyte macrophage-colony stimulating factor) on human osteoclast formation from circulating cells. Bone. 2001 Mar;28(3):261-7.). Therefore, we investigated the effect of different kinds of immunostimulatory ODNs in the osteogenetic process.

Table 2 shows that the best stimulation of the osteogenesis was obtained using immunostimulatory ODNs of the PyNTTTTGT class (Elias F, Flo J, Lopez RA, Zorzopulos J, Montaner A, Rodriguez JM. Strong cytosine-guanosine-independent immunostimulation in humans and other primates by synthetic oligodeoxynucleotides with PyNTTTTGT motifs. J Immunol. 2003 Oct 1;171(7)3697-704.).

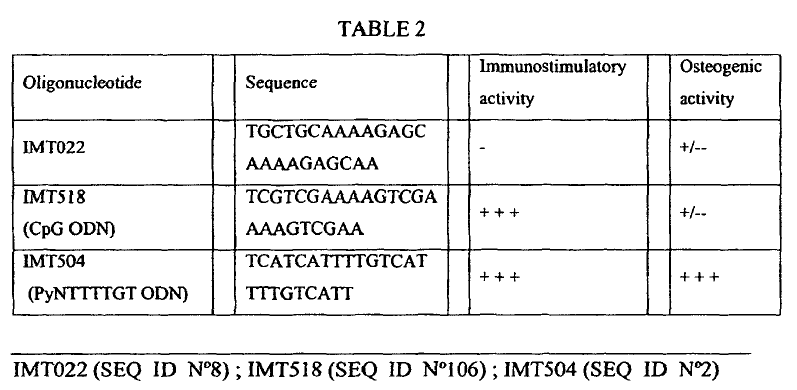

These results indicate that ODNs with strong immunostimulatory activity are not necessarily strong stimulators of the osteogesis. However, the most active ODNs in osteogenesis are strongly immunostimulatory.

SEQUENCE LISTING

<110>  IMMUNOTECH S.A.

<120>  Osteogenic oligonucleotides and uses thereof

<130>  04ST44E

<140>  EP04077653.6
<141>  2004-09-27

<160>  128

<170>  PatentIn version 3.3

<210>  1
<211>  24
<212>  DNA
<213>  Artificial

<220>
<223>  Synthetic Construct

<400>  1
tgctgctttt gtgcttttgt gctt                                              24


<210>  2
<211>  24
<212>  DNA
<213>  Artificial

<220>
<223>  Synthetic Construct

<400>  2
tcatcatttt gtcattttgt catt                                              24


<210>  3
<211>  24
<212>  DNA
<213>  Artificial

<220>
<223>  Synthetic Construct

<400>  3
tcctcctttt gtccttttgt cctt                                              24


<210>  4
<211>  24
<212>  DNA
<213>  Artificial

<220>
<223>  Synthetic Construct

<400>  4
   tcttcttttt gtctttttgt cttt                                              24


<210>  5
<211>  24
<212>  DNA
<213>  Artificial

<220>
<223>  Synthetic Construct

<400>  5
tagtagtttt gtagttttgt agtt                                              24


<210>  6
<211>  24
<212>  DNA
<213>  Artificial

<220>
<223>  Synthetic Construct

<400>  6
tggtggtttt gtggttttgt ggtt                                              24


<210>  7
<211>  24
<212>  DNA
<213>  Artificial

<220>
<223>  Synthetic Construct

<400>  7
ttgttgtttt gttgttttgt tgtt                                              24


<210>  8
<211>  24
<212>  DNA
<213>  Artificial

<220>
<223>  Synthetic Construct

<400>  8
tgctgcaaaa gagcaaaaga gcaa                                              24


<210>  9
<211>  24
<212>  DNA
<213>  Artificial

<220>
<223>  Synthetic Construct

<400>  9
tgctgccccc gcgcccccgc gccc                                              24


<210>  10
<211>  24
<212>  DNA
<213>  Artificial

<220>
<223>  Synthetic Construct

<400>  10
tcattttttt gtttttttgt catt                                              24


<210>  11

<211>  24
<212>  DNA
<213>  Artificial

<220>
<223>  Synthetic Construct

<400>  11
tcattgtttt gttgttttgt catt                                              24


<210>  12
<211>  24
<212>  DNA
<213>  Artificial

<220>
<223>  Synthetic Construct

<400>  12
tcattctttt gttcttttgt catt                                              24


<210>  13
<211>  24
<212>  DNA
<213>  Artificial

<220>
<223>  Synthetic Construct

<400>  13
aaaaaactaa aaaaaactaa aaaa                                              24


<210>  14
<211>  24
<212>  DNA
<213>  Artificial

<220>
<223>  Synthetic Construct

<400>  14
tcataatttt gtaattttgt catt                                              24


<210>  15
<211>  24
<212>  DNA
<213>  Artificial

<220>
<223>  Synthetic Construct

<400>  15
tcattatttt gttattttgt catt                                              24


<210>  16
<211>  24
<212>  DNA
<213>  Artificial

<220>
<223>  Synthetic Construct

<400>  16

tcatgatttt gtgattttgt catt                                          24

<210>  17
<211>  24
<212>  DNA
<213>  Artificial

<220>
<223>  Synthetic Construct

<400>  17
tcatcctttt gtccttttgt catt                                          24

<210>  18
<211>  24
<212>  DNA
<213>  Artificial

<220>
<223>  Synthetic Construct

<400>  18
   tcatcttttt gtctttttgt catt                                           24

<210>  19
<211>  24
<212>  DNA
<213>  Artificial

<220>
<223>  Synthetic Construct

<400>  19
tttttttttt tttttttttt tttt                                          24

<210>  20
<211>  24
<212>  DNA
<213>  Artificial

<220>
<223>  Synthetic Construct

<400>  20
cattttgttt tttttttttt tttt                                          24

<210>  21
<211>  24
<212>  DNA
<213>  Artificial

<220>
<223>  Synthetic Construct

<400>  21
ttcattttgt tttttttttt tttt                                          24

<210>  22
<211>  24
<212>  DNA
<213>  Artificial

<220>
<223>  Synthetic Construct

<400>  22
ttttcatttt gttttttttt tttt                                              24


<210>  23
<211>  24
<212>  DNA
<213>  Artificial

<220>
<223>  Synthetic Construct

<400>  23
tttttcatt ttgttttttt tttt                                               24


<210>  24
<211>  24
<212>  DNA
<213>  Artificial

<220>
<223>  Synthetic Construct

<400>  24
ttttttttca ttttgttttt tttt                                              24


<210>  25
<211>  24
<212>  DNA
<213>  Artificial

<220>
<223>  Synthetic Construct

<400>  25
tttttttttt cattttgttt tttt                                              24


<210>  26
<211>  24
<212>  DNA
<213>  Artificial

<220>
<223>  Synthetic Construct

<400>  26
tttttttttt ttcattttgt tttt                                              24


<210>  27
<211>  24
<212>  DNA
<213>  Artificial

<220>
<223>  Synthetic Construct

<400>  27
tttttttttt ttttcatttt gttt                                              24


<210>  28

<211>  24
<212>  DNA
<213>  Artificial

<220>
<223>  Synthetic Construct

<400>  28
tttttttttt ttttttcatt ttgt                                              24


<210>  29
<211>  24
<212>  DNA
<213>  Artificial

<220>
<223>  Synthetic Construct

<400>  29
ttttcatttt gtcattttgt tttt                                              24


<210>  30
<211>  24
<212>  DNA
<213>  Artificial

<220>
<223>  Synthetic Construct

<400>  30
tcatcaattt gtcaatttgt catt                                              24


<210>  31
<211>  24
<212>  DNA
<213>  Artificial

<220>
<223>  Synthetic Construct

<400>  31
tcatcatatt gtcatattgt catt                                              24


<210>  32
<211>  24
<212>  DNA
<213>  Artificial

<220>
<223>  Synthetic Construct

<400>  32
tcatcattat gtcattatgt catt                                              24


<210>  33
<211>  24
<212>  DNA
<213>  Artificial

<220>
<223>  Synthetic Construct

<400>  33

tcatcattta gtcatttagt catt                                                24

<210>  34
<211>  24
<212>  DNA
<213>  Artificial

<220>
<223>  Synthetic Construct

<400>  34
tcatcatttt atcattttat catt                                                24

<210>  35
<211>  24
<212>  DNA
<213>  Artificial

<220>
<223>  Synthetic Construct

<400>  35
tcatcatttt ttcatttttt catt                                                24

<210>  36
<211>  24
<212>  DNA
<213>  Artificial

<220>
<223>  Synthetic Construct

<400>  36
tcatcatttt ctcattttct catt                                                24

<210>  37
<211>  24
<212>  DNA
<213>  Artificial

<220>
<223>  Synthetic Construct

<400>  37
tcatcatttt gacattttga catt                                                24

<210>  38
<211>  24
<212>  DNA
<213>  Artificial

<220>
<223>  Synthetic Construct

<400>  38
tcatcattta gacatttaga catt                                                24

<210>  39
<211>  24
<212>  DNA
<213>  Artificial

<220>
<223>  Synthetic Construct

<400>  39
tcatcattat gacattatga catt                                              24


<210>  40
<211>  24
<212>  DNA
<213>  Artificial

<220>
<223>  Synthetic Construct

<400>  40
tcatcatatt gacatattga catt                                              24


<210>  41
<211>  24
<212>  DNA
<213>  Artificial

<220>
<223>  Synthetic Construct

<400>  41
tcatcattaa gacattaaga catt                                              24


<210>  42
<211>  24
<212>  DNA
<213>  Artificial

<220>
<223>  Synthetic Construct

<400>  42
tcatcatata gacatataga catt                                              24


<210>  43
<211>  24
<212>  DNA
<213>  Artificial

<220>
<223>  Synthetic Construct

<400>  43
tcatcataat gacataatga catt                                              24


<210>  44
<211>  24
<212>  DNA
<213>  Artificial

<220>
<223>  Synthetic Construct

<400>  44
tcatcataaa gacataaaga catt                                              24


<210>  45

<211>  24
<212>  DNA
<213>  Artificial

<220>
<223>  Synthetic Construct

<400>  45
tcatcaaaaa gacaaaaaga catt                                              24


<210>  46
<211>  24
<212>  DNA
<213>  Artificial

<220>
<223>  Synthetic Construct

<400>  46
acatcatttt gtcattttgt catt                                              24


<210>  47
<211>  24
<212>  DNA
<213>  Artificial

<220>
<223>  Synthetic Construct

<400>  47
ccatcatttt gtcattttgt catt                                              24


<210>  48
<211>  24
<212>  DNA
<213>  Artificial

<220>
<223>  Synthetic Construct

<400>  48
gcatcatttt gtcattttgt catt                                              24


<210>  49
<211>  24
<212>  DNA
<213>  Artificial

<220>
<223>  Synthetic Construct

<400>  49
taatcatttt gtcattttgt catt                                              24


<210>  50
<211>  24
<212>  DNA
<213>  Artificial

<220>
<223>  Synthetic Construct

<400>  50

ttatcatttt gtcattttgt catt                                              24


<210>  51
<211>  24
<212>  DNA
<213>  Artificial

<220>
<223>  Synthetic Construct

<400>  51
tgatcatttt gtcattttgt catt                                              24


<210>  52
<211>  24
<212>  DNA
<213>  Artificial

<220>
<223>  Synthetic Construct

<400>  52
tcctcatttt gtcattttgt catt                                              24


<210>  53
<211>  24
<212>  DNA
<213>  Artificial

<220>
<223>  Synthetic Construct

<400>  53
tcttcatttt gtcattttgt catt                                              24


<210>  54
<211>  24
<212>  DNA
<213>  Artificial

<220>
<223>  Synthetic Construct

<400>  54
tcgtcatttt gtcattttgt catt                                              24


<210>  55
<211>  24
<212>  DNA
<213>  Artificial

<220>
<223>  Synthetic Construct

<400>  55
tcaacatttt gtcattttgt catt                                              24


<210>  56
<211>  24
<212>  DNA
<213>  Artificial

<220>
<223>  Synthetic Construct

<400>  56
tcaccatttt gtcattttgt catt                                              24


<210>  57
<211>  24
<212>  DNA
<213>  Artificial

<220>
<223>  Synthetic Construct

<400>  57
tcagcatttt gtcattttgt catt                                              24


<210>  58
<211>  24
<212>  DNA
<213>  Artificial

<220>
<223>  Synthetic Construct

<400>  58
tcatcatttt gtcattttgt aatt                                              24


<210>  59
<211>  24
<212>  DNA
<213>  Artificial

<220>
<223>  Synthetic Construct

<400>  59
tcatcatttt gtcattttgt tatt                                              24


<210>  60
<211>  24
<212>  DNA
<213>  Artificial

<220>
<223>  Synthetic Construct

<400>  60
tcatcatttt gtcattttgt gatt                                              24


<210>  61
<211>  24
<212>  DNA
<213>  Artificial

<220>
<223>  Synthetic Construct

<400>  61
tcatcatttt gtcattttgt cctt                                              24


<210>  62

<211>  24
<212>  DNA
<213>  Artificial

<220>
<223>  Synthetic Construct

<400>  62
tcatcatttt gtcattttgt cttt                                              24


<210>  63
<211>  24
<212>  DNA
<213>  Artificial

<220>
<223>  Synthetic Construct

<400>  63
tcatcatttt gtcattttgt cgtt                                              24


<210>  64
<211>  24
<212>  DNA
<213>  Artificial

<220>
<223>  Synthetic Construct

<400>  64
tcatcatttt gtcattttgt caat                                              24


<210>  65
<211>  24
<212>  DNA
<213>  Artificial

<220>
<223>  Synthetic Construct

<400>  65
tcatcatttt gtcattttgt cact                                              24


<210>  66
<211>  24
<212>  DNA
<213>  Artificial

<220>
<223>  Synthetic Construct

<400>  66
tcatcatttt gtcattttgt cagt                                              24


<210>  67
<211>  24
<212>  DNA
<213>  Artificial

<220>
<223>  Synthetic Construct

<400>  67

tcatcatttt gtcattttgt cata                                              24

<210>  68
<211>  24
<212>  DNA
<213>  Artificial

<220>
<223>  Synthetic Construct

<400>  68
tcatcatttt gtcattttgt catc                                              24

<210>  69
<211>  24
<212>  DNA
<213>  Artificial

<220>
<223>  Synthetic Construct

<400>  69
tcatcatttt gtcattttgt catg                                              24

<210>  70
<211>  12
<212>  DNA
<213>  Artificial

<220>
<223>  Synthetic Construct

<400>  70
ttttcatttt gt                                                           12

<210>  71
<211>  16
<212>  DNA
<213>  Artificial

<220>
<223>  Synthetic Construct

<400>  71
ttttcatttt gttttt                                                       16

<210>  72
<211>  20
<212>  DNA
<213>  Artificial

<220>
<223>  Synthetic Construct

<400>  72
ttttcatttt gttttttttt                                                   20

<210>  73
<211>  24
<212>  DNA
<213>  Artificial

<220>
<223>  Synthetic Construct

<400>  73
tttttttttt ttcattttgt tttt                                              24


<210>  74
<211>  28
<212>  DNA
<213>  Artificial

<220>
<223>  Synthetic Construct

<400>  74
ttttcatttt gttttttttt tttttttt                                          28


<210>  75
<211>  24
<212>  DNA
<213>  Artificial

<220>
<223>  Synthetic Construct

<400>  75
tcatcaattg gtcaattggt catt                                              24


<210>  76
<211>  24
<212>  DNA
<213>  Artificial

<220>
<223>  Synthetic Construct

<400>  76
tcatcaaatg gtcaaatggt catt                                              24


<210>  77
<211>  24
<212>  DNA
<213>  Artificial

<220>
<223>  Synthetic Construct

<400>  77
tcatcaactg gtcaactggt catt                                              24


<210>  78
<211>  24
<212>  DNA
<213>  Artificial

<220>
<223>  Synthetic Construct

<400>  78
tcatcaagtg gtcaagtggt catt                                              24


<210>  79

<211>  24
<212>  DNA
<213>  Artificial

<220>
<223>  Synthetic Construct

<400>  79
tcatcaatag gtcaataggt catt                                              24


<210>  80
<211>  24
<212>  DNA
<213>  Artificial

<220>
<223>  Synthetic Construct

<400>  80
tcatcaatcg gtcaatcggt catt                                              24


<210>  81
<211>  24
<212>  DNA
<213>  Artificial

<220>
<223>  Synthetic Construct

<400>  81
tcatcaatgg gtcaatgggt catt                                              24


<210>  82
<211>  24
<212>  DNA
<213>  Artificial

<220>
<223>  Synthetic Construct

<400>  82
tcatcattat gtcattatgt catt                                              24


<210>  83
<211>  24
<212>  DNA
<213>  Artificial

<220>
<223>  Synthetic Construct

<400>  83
tcatcattct gtcattctgt catt                                              24


<210>  84
<211>  24
<212>  DNA
<213>  Artificial

<220>
<223>  Synthetic Construct

<400>  84

tcatcattgt gtcattgtgt catt                                              24

<210>  85
<211>  24
<212>  DNA
<213>  Artificial

<220>
<223>  Synthetic Construct

<400>  85
tcatcattta gtcatttagt catt                                              24

<210>  86
<211>  24
<212>  DNA
<213>  Artificial

<220>
<223>  Synthetic Construct

<400>  86
tcatcatttc gtcatttcgt catt                                              24

<210>  87
<211>  24
<212>  DNA
<213>  Artificial

<220>
<223>  Synthetic Construct

<400>  87
tcatcatttg gtcatttggt catt                                              24

<210>  88
<211>  24
<212>  DNA
<213>  Artificial

<220>
<223>  Synthetic Construct

<400>  88
tcatcatttt gacattttga catt                                              24

<210>  89
<211>  24
<212>  DNA
<213>  Artificial

<220>
<223>  Synthetic Construct

<400>  89
tcatcatttt gccattttgc catt                                              24

<210>  90
<211>  24
<212>  DNA
<213>  Artificial

<220>
<223>  Synthetic Construct

<400>  90
tcatcatttt ggcattttgg catt                                              24


<210>  91
<211>  24
<212>  DNA
<213>  Artificial

<220>
<223>  Synthetic Construct

<400>  91
tgctgctttt gtgcttttgt gctt                                              24


<210>  92
<211>  24
<212>  DNA
<213>  Artificial

<220>
<223>  Synthetic Construct

<400>  92
tcatcacttt ggcactttgg catt                                              24


<210>  93
<211>  24
<212>  DNA
<213>  Artificial

<220>
<223>  Synthetic Construct

<400>  93
tcatcagttt ggcagtttgg catt                                              24


<210>  94
<211>  24
<212>  DNA
<213>  Artificial

<220>
<223>  Synthetic Construct

<400>  94
tcatcatatt ggcatattgg catt                                              24


<210>  95
<211>  24
<212>  DNA
<213>  Artificial

<220>
<223>  Synthetic Construct

<400>  95
tcatcatctt ggcatcttgg catt                                              24


<210>  96

<211>  24
<212>  DNA
<213>  Artificial

<220>
<223>  Synthetic Construct

<400>  96
tcatcatgtt ggcatgttgg catt                                              24


<210>  97
<211>  24
<212>  DNA
<213>  Artificial

<220>
<223>  Synthetic Construct

<400>  97
tgctgccccc gcgcccccgc gccc                                              24


<210>  98
<211>  24
<212>  DNA
<213>  Artificial

<220>
<223>  Synthetic Construct

<400>  98
ggggtttttt tttttttttt tttt                                              24


<210>  99
<211>  24
<212>  DNA
<213>  Artificial

<220>
<223>  Synthetic Construct

<400>  99
   ggggtctttt tttctttttt tctt                                               24


<210>  100
<211>  24
<212>  DNA
<213>  Artificial

<220>
<223>  Synthetic Construct

<400>  100
tgctgccccc gcgcccccgc gccc                                              24


<210>  101
<211>  24
<212>  DNA
<213>  Artificial

<220>
<223>  Synthetic Construct

<400>  101

ggggactttt atacttttat actt                                          24


<210>  102
<211>  24
<212>  DNA
<213>  Artificial

<220>
<223>  Synthetic Construct

<400>  102
ggggtatttt tttattttt tatt                                          24


<210>  103
<211>  24
<212>  DNA
<213>  Artificial

<220>
<223>  Synthetic Construct

<400>  103
gggggctttt gggcttttgg gcgg                                          24


<210>  104
<211>  24
<212>  DNA
<213>  Artificial

<220>
<223>  Synthetic Construct

<400>  104
gggggcttta gtgctttagt gctt                                          24


<210>  105
<211>  24
<212>  DNA
<213>  Artificial

<220>
<223>  Synthetic Construct

<400>  105
gggggctaat gtgctaatgt gctt                                          24


<210>  106
<211>  24
<212>  DNA
<213>  Artificial

<220>
<223>  Synthetic Construct

<400>  106
tcgtcgaaaa gtcgaaaagt cgaa                                          24


<210>  107
<211>  24
<212>  DNA
<213>  Artificial

<220>
<223>  Synthetic Construct

<400>  107
gggggtcttt ttttcttttt tttt                                              24


<210>  108
<211>  24
<212>  DNA
<213>  Artificial

<220>
<223>  Synthetic Construct

<400>  108
   tttttctttt ttttcttttt tggg                                              24


<210>  109
<211>  24
<212>  DNA
<213>  Artificial

<220>
<223>  Synthetic Construct

<400>  109
aaaaatcttt ttttcttttt tttt                                              24


<210>  110
<211>  24
<212>  DNA
<213>  Artificial

<220>
<223>  Synthetic Construct

<400>  110
tgctgctttt atgcttttat gctt                                              24


<210>  111
<211>  24
<212>  DNA
<213>  Artificial

<220>
<223>  Synthetic Construct

<400>  111
tcatcattct gtcattctgt catt                                              24


<210>  112
<211>  24
<212>  DNA
<213>  Artificial

<220>
<223>  Synthetic Construct

<400>  112
   tttttctttt ttttcttttt tttt                                              24


<210>  113

<211>  24
<212>  DNA
<213>  Artificial

<220>
<223>  Synthetic Construct

<400>  113
tgctgctttt ctgcttttct gctt                                              24


<210>  114
<211>  24
<212>  DNA
<213>  Artificial

<220>
<223>  Synthetic Construct

<400>  114
tttttctttt tctttttttt tttt                                              24


<210>  115
<211>  24
<212>  DNA
<213>  Artificial

<220>
<223>  Synthetic Construct

<400>  115
tttttccttt tttcctttttt tttt                                              24


<210>  116
<211>  24
<212>  DNA
<213>  Artificial

<220>
<223>  Synthetic Construct

<400>  116
ccccctcttt ttttcttttt tttt                                              24


<210>  117
<211>  24
<212>  DNA
<213>  Artificial

<220>
<223>  Synthetic Construct

<400>  117
tttttctttt ttcttttttt tttt                                              24


<210>  118
<211>  24
<212>  DNA
<213>  Artificial

<220>
<223>  Synthetic Construct

<400>  118
   ttttttcttt ttttcttttt tccc                                                24

<210>  119
<211>  24
<212>  DNA
<213>  Artificial

<220>
<223>  Synthetic Construct

<400>  119
   ttttttcttt ttctcttttt ctct                                                24

<210>  120
<211>  24
<212>  DNA
<213>  Artificial

<220>
<223>  Synthetic Construct

<400>  120
tttttgcttt tttgcttttt tttt                                                24

<210>  121
<211>  24
<212>  DNA
<213>  Artificial

<220>
<223>  Synthetic Construct

<400>  121
   tttttacttt tttacttttt tttt                                                24

<210>  122
<211>  23
<212>  DNA
<213>  Artificial

<220>
<223>  Synthetic Construct

<400>  122
cataattttg taattttgtc att                                                23

<210>  123
<211>  24
<212>  DNA
<213>  Artificial

<220>
<223>  Synthetic Construct

<400>  123
   ttttttcttt ttttcttttt taaa                                                24

<210>  124
<211>  24
<212>  DNA
<213>  Artificial

<220>
<223>  Synthetic Construct

<400>  124
tttttctttt tttcttttt tttt                                              24


<210>  125
<211>  24
<212>  DNA
<213>  Artificial

<220>
<223>  Synthetic Construct

<400>  125
tttttttttt ttcattttgt gggg                                              24


<210>  126
<211>  24
<212>  DNA
<213>  Artificial

<220>
<223>  Synthetic Construct

<400>  126
tttttttttt ttcattttgt tttg                                              24


<210>  127
<211>  24
<212>  DNA
<213>  Artificial

<220>
<223>  Synthetic Construct

<400>  127
gggttttttt ttcattttgt tttt                                              24


<210>  128
<211>  24
<212>  DNA
<213>  Artificial

<220>
<223>  Synthetic Construct

<400>  128
gttttttttt ttcattttgt tttg                                              24

### Annex to the application documents - subsequently filed sequences listing

SEQUENCE LISTING

(1) GENERAL INFORMATION:

(i) APPLICANT: IMMUNOTECH S.A.

(ii) TITLE OF INVENTION: Osteogenic oligonucleotides and uses thereof

(iii) NUMBER OF SEQUENCES: 128

(iv) CORRESPONDENCE ADDRESS:

(A) ADDRESSEE: Dragotti & Associati

(B) STREET: Galleria San Babila 4C

(C) CITY: Milano

(D) STATE:

(E) COUNTRY: Italy

(F) ZIP: 20122

(v) COMPUTER READABLE FORM:

(A) MEDIUM TYPE: Floppy disk

(B) COMPUTER: IBM PC compatible

(vi) CURRENT APPLICATION DATA:

(A) APPLICATION NUMBER:

(B) FILING DATE:

(C) CLASSIFICATION:

(vii) ATTORNEY/AGENT INFORMATION:

(A) NAME: Roberto Pistolesi

(B) REGISTRATION NUMBER: 94880

(C) REFERENCE/DOCKET NUMBER: 04st44e

(viii) TELECOMMUNICATION INFORMATION:

(A) TELEFONE: 02 799340

(B) TELEFAX: 02784427

(2) INFORMATION FOR SEQ ID N°1 (IMT 021):

(i) SEQUENCE CHARACTERISTICS:

(A) LENGTH: 24 base pairs

(B) TYPE: nucleic acid

(C) STRANDEDNESS: single
(D) TOPOLOGY: linear
(ii) MOLECULE TYPE: DNA
(iii) SEQUENCE DESCRIPTION: SEQ ID Nº 1:
TGCTGCTTTTGTGCTTTTGTGCTT
(3) INFORMATION FOR SEQ ID Nº2 (IMT 504):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID Nº 2:
TCATCATTTTGTCATTTTGTCATT
(4) INFORMATION FOR SEQ ID Nº3 (IMT 505):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID Nº 3:
TCCTCCTTTTGTCCTTTTGTCCTT
(5) INFORMATION FOR SEQ ID Nº4 (IMT 506):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA

(iii) SEQUENCE DESCRIPTION: SEQ ID N° 4:

TCTTCTTTTTGTCTTTTTGTCTTT

(6) INFORMATION FOR SEQ ID N°5 (IMT 501):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 5:

TAGTAGTTTTGTAGTTTTGTAGTT

(7) INFORMATION FOR SEQ ID N°6 (IMT 502):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 6:

TGGTGGTTTTGTGGTTTTGTGGTT

(8) INFORMATION FOR SEQ ID N°7 (IMT 503):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 7:

TTGTTGTTTTGTTGTTTTGTTGTT

(9) INFORMATION FOR SEQ ID N°8 (IMT 022):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 8:

TGCTGCAAAAGAGCAAAAGAGCAA

(10) INFORMATION FOR SEQ ID N°9 (IMT 023):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 9:

TGCTGCCCCCGCGCCCCCGCGCCC

(11) INFORMATION FOR SEQ ID N°10 (IMT 197):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 10:

TCATTTTTTTGTTTTTTTGTCATT

(12) INFORMATION FOR SEQ ID N°11 (IMT 198):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 11:

TCATTGTTTTGTTGTTTTGTCATT

(13) INFORMATION FOR SEQ ID N°12 (IMT 199):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 12:

TCATTCTTTTGTTCTTTTGTCATT

(14) INFORMATION FOR SEQ ID N°13 (IMT 509):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 13.

AAAAAACTAAAAAAAACTAAAAAA

(15) INFORMATION FOR SEQ ID N°14 (IMT 531):

(i) SEQUENCE CHARACTERISTICS:

(A) LENGTH: 24 base pairs

(B) TYPE: nucleic acid

(C) STRANDEDNESS: single

(D) TOPOLOGY: linear

(ii)	MOLECULE TYPE: DNA
(iii)	SEQUENCE DESCRIPTION: SEQ ID N° 14:
TCATAATTTTGTAATTTTGTCATT
(16) INFORMATION FOR SEQ ID N°15 (IMT 532):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 15:
TCATTATTTTGTTATTTTGTCATT
(17) INFORMATION FOR SEQ ID N°16 (IMT 533):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 16:
TCATGATTTTGTGATTTTGTCATT
(18) INFORMATION FOR SEQ ID N°17 (IMT 534):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 17:
TCATCCTTTTGTCCTTTTGTCATT

(19) INFORMATION FOR SEQ ID N°18 (IMT 535):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE. DNA

(iii) SEQUENCE DESCRIPTION: SEQ ID N° 18:

TCATCTTTTTGTCTTTTTGTCATT

(20) INFORMATION FOR SEQ ID N°19 (IMT 053):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 19:

TTTTTTTTTTTTTTTTTTTTTTTT

(21) INFORMATION FOR SEQ ID N°20 (IMT 173):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 20:

CATTTTGTTTTTTTTTTTTTTTTT

(22) INFORMATION FOR SEQ ID N°21 (IMT 174):

(i)	SEQUENCE CHARACTERISTICS:

(A) LENGTH: 24 base pairs

(B) TYPE: nucleic acid

(C) STRANDEDNESS: single

(D) TOPOLOGY: linear

(ii)	MOLECULE TYPE: DNA

(iii)	SEQUENCE DESCRIPTION: SEQ ID N° 21:

TTCATTTTGTTTTTTTTTTTTTTT

(23) INFORMATION FOR SEQ ID N°22 (IMT 175):

(i)	SEQUENCE CHARACTERISTICS:

(A) LENGTH: 24 base pairs

(B) TYPE: nucleic acid

(C) STRANDEDNESS: single

(D) TOPOLOGY: linear

(ii)	MOLECULE TYPE: DNA

(iii)	SEQUENCE DESCRIPTION: SEQ ID N° 22:

TTTTCATTTTGTTTTTTTTTTTTT

(24) INFORMATION FOR SEQ ID N°23 (IMT 176):

(i)	SEQUENCE CHARACTERISTICS:

(A) LENGTH: 24 base pairs

(B) TYPE: nucleic acid

(C) STRANDEDNESS: single

(D) TOPOLOGY: linear

(ii)	MOLECULE TYPE: DNA

(iii)	SEQUENCE DESCRIPTION: SEQ ID N° 23:

TTTTTTCATTTTGTTTTTTTTTTT

(25) INFORMATION FOR SEQ ID N°24 (IMT 177):

(i)	SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 24:

TTTTTTTTCATTTTGTTTTTTTTT

(26) INFORMATION FOR SEQ ID N°25 (IMT 178):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 25:

TTTTTTTTTTCATTTTGTTTTTTT

(27) INFORMATION FOR SEQ ID N°26 (IMT 179):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 26:

TTTTTTTTTTTTCATTTTGTTTTT

(28) INFORMATION FOR SEQ ID N°27(IMT 180):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 27:

TTTTTTTTTTTTTTCATTTTGTTT

(29) INFORMATION FOR SEQ ID N°28 (IMT 181):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 28:

TTTTTTTTTTTTTTTTCATTTTGT

(30) INFORMATION FOR SEQ ID N°29 (IMT 182):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 29:

TTTTCATTTTGTCATTTTGTTTTT

(31) INFORMATION FOR SEQ ID N°30 (IMT 537):

(i) SEQUENCE CHARACTERISTICS:

(A) LENGTH: 24 base pairs

(B) TYPE: nucleic acid

(C) STRANDEDNESS: single

(D) TOPOLOGY: linear

(ii) MOLECULE TYPE: DNA

(iii) SEQUENCE DESCRIPTION: SEQ ID N° 30:

TCATCAATTTGTCAATTTGTCATT

(32) INFORMATION FOR SEQ ID N°31 (IMT 538):
(i) SEQUENCE CHARACTERISTICS:
(A) LENGTH 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 31:
TCATCATATTGTCATATTGTCATT

(33) INFORMATION FOR SEQ ID N°32 (IMT 539):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 32:
TCATCATTATGTCATTATGTCATT

(34) INFORMATION FOR SEQ ID N°33 (IMT 540):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 33:
TCATCATTTAGTCATTTAGTCATT

(35) INFORMATION FOR SEQ ID N°34 (IMT 541):
(i) SEQUENCE CHARACTERISTICS:
(A) LENGTH: 24 base pairs

(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 34:
TCATCATTTTATCATTTTATCATT

(36) INFORMATION FOR SEQ ID N°35 (IMT 542):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 35:
TCATCATTTTTTCATTTTTTCATT

(37) INFORMATION FOR SEQ ID N°36 (IMT 543):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 36:
TCATCATTTTCTCATTTTCTCATT

(38) INFORMATION FOR SEQ ID N°37 (IMT 544):
(i) SEQUENCE CHARACTERISTICS:
(A) LENGTH: 24 base pairs
(B) TYPE: nucleic acid
(C) STRANDEDNESS: single
(D) TOPOLOGY: linear

(ii)	MOLECULE TYPE: DNA
(iii)	SEQUENCE DESCRIPTION: SEQ ID N° 37:
TCATCATTTTGACATTTTGACATT

(39) INFORMATION FOR SEQ ID N°38 (IMT 545):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 38:
TCATCATTTAGACATTTAGACATT

(40) INFORMATION FOR SEQ ID N°39 (IMT 546):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 39:
TCATCATTATGACATTATGACATT

(41) INFORMATION FOR SEQ ID N°40 (IMT 547):
(i)	SEQUENCE CHARACTERISTICS:
(A) LENGTH: 24 base pairs
(B) TYPE: nucleic acid
(C) STRANDEDNESS: single
(D) TOPOLOGY: linear
(ii)	MOLECULE TYPE: DNA
(iii)	SEQUENCE DESCRIPTION: SEQ ID N° 40:
TCATCATATTGACATATTGACATT

(42) INFORMATION FOR SEQ ID N°41 (IMT 548):
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA
(iii) SEQUENCE DESCRIPTION: SEQ ID N° 41:
TCATCATTAAGACATTAAGACATT

(43) INFORMATION FOR SEQ ID N°42 (IMT 549):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 42:
TCATCATATAGACATATAGACATT

(44) INFORMATION FOR SEQ ID N°43 (IMT 550):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 43:
TCATCATAATGACATAATGACATT

(45) INFORMATION FOR SEQ ID N°44 (IMT 551):

(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 44:
TCATCATAAAGACATAAAGACATT

(46) INFORMATION FOR SEQ ID N°45 (IMT 552):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 45:
TCATCAAAAAGACAAAAAGACATT

(47) INFORMATION FOR SEQ ID N°46 (IMT 559):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 46:
ACATCATTTTGTCATTTTGTCATT

(48) INFORMATION FOR SEQ ID N°47 (IMT 560):
(A) SEQUENCE CHARACTERISTICS:
(A) LENGTH: 24 base pairs

(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 47:
CCATCATTTTGTCATTTTGTCATT

(49) INFORMATION FOR SEQ ID N°48 (IMT 561):
(A) SEQUENCE CHARACTERISTICS:
(A) LENGTH: 24 base pairs
(B) TYPE: nucleic acid
(C) STRANDEDNESS: single
(D) TOPOLOGY: linear
(B) MOLECULE TYPE: DNA
(C) SEQUENCE DESCRIPTION: SEQ ID N° 48:
GCATCATTTTGTCATTTTGTCATT

(50) INFORMATION FOR SEQ ID N°49 (IMT 562):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 49:
TAATCATTTTGTCATTTTGTCATT

(51) INFORMATION FOR SEQ ID N°50 (IMT 563):
I. SEQUENCE CHARACTERISTICS:
(A) LENGTH: 24 base pairs
(B) TYPE: nucleic acid
(C) STRANDEDNESS: single

(D) TOPOLOGY: linear
II. MOLECULE TYPE: DNA
III. SEQUENCE DESCRIPTION: SEQ ID N° 50:
TTATCATTTTGTCATTTTGTCATT
(52) INFORMATION FOR SEQ ID N°51 (IMT 564):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 51:
TGATCATTTTGTCATTTTGTCATT
(53) INFORMATION FOR SEQ ID N°52 (IMT 565):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 52:
TCCTCATTTTGTCATTTTGTCATT
(54) INFORMATION FOR SEQ ID N°53 (IMT 566):
I. SEQUENCE CHARACTERISTICS:
(A) LENGTH: 24 base pairs
(B) TYPE: nucleic acid
(C) STRANDEDNESS: single
(D) TOPOLOGY: linear
II MOLECULE TYPE: DNA
III. SEQUENCE DESCRIPTION: SEQ ID N° 53:

TCTTCATTTTGTCATTTTGTCATT

(55) INFORMATION FOR SEQ ID N°54 (IMT 567):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 54:

TCGTCATTTTGTCATTTTGTCATT

(56) INFORMATION FOR SEQ ID N°55 (IMT 568):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 55:

TCAACATTTTGTCATTTTGTCATT

(57) INFORMATION FOR SEQ ID N°56 (IMT 569):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 56:

TCACCATTTTGTCATTTTGTCATT

(58) INFORMATION FOR SEQ ID N°57 (IMT 570):

I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 57:
TCAGCATTTTGTCATTTTGTCATT

(59) INFORMATION FOR SEQ ID N°58 (IMT 571):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 58:
TCATCATTTTGTCATTTTGTAATT

(60) INFORMATION FOR SEQ ID N°59 (IMT 572):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 59:
TCATCATTTTGTCATTTTGTTATT

(61) INFORMATION FOR SEQ ID N°60 (IMT 573):
I. SEQUENCE CHARACTERISTICS:
(A) LENGTH: 24 base pairs
(B) TYPE: nucleic acid

(C) STRANDEDNESS: single

(D) TOPOLOGY: linear

II. MOLECULE TYPE: DNA

III. SEQUENCE DESCRIPTION: SEQ ID N° 60:

TCATCATTTTGTCATTTTGTGATT

(62) INFORMATION FOR SEQ ID N°61 (IMT 574):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 61:

TCATCATTTTGTCATTTTGTCCTT

(63) INFORMATION FOR SEQ ID N°62 (IMT 575):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 62:

TCATCATTTTGTCATTTTGTCTTT

(64) INFORMATION FOR SEQ ID N°63 (IMT 576):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 63:

TCATCATTTTGTCATTTTGTCGTT

(65) INFORMATION FOR SEQ ID N°64 (IMT 577):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III SEQUENCE DESCRIPTION: SEQ ID N° 64:

TCATCATTTTGTCATTTTGTCAAT

(66) INFORMATION FOR SEQ ID N°65 (IMT 578):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 65:

TCATCATTTTGTCATTTTGTCACT

(67) INFORMATION FOR SEQ ID N°66 (IMT 579):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 66:

TCATCATTTTGTCATTTTGTCAGT

(68) INFORMATION FOR SEQ ID N°67 (IMT 580):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III SEQUENCE DESCRIPTION: SEQ ID N° 67:
TCATCATTTTGTCATTTTGTCATA

(69) INFORMATION FOR SEQ ID N°68 (IMT 581):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 68:
TCATCATTTTGTCATTTTGTCATC

(70) INFORMATION FOR SEQ ID N°69 (IMT 582):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 69:
TCATCATTTTGTCATTTTGTCATG

(71) INFORMATION FOR SEQ ID N°70 (IMT 187):
I. SEQUENCE CHARACTERISTICS:
(A) LENGTH: 12 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 70:

TTTTCATTTTGT

(72) INFORMATION FOR SEQ ID N°71 (IMT 188):

I. SEQUENCE CHARACTERISTICS:

(A) LENGTH: 16 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 71:

TTTTCATTTTGTTTTT

(73) INFORMATION FOR SEQ ID N°72 (IMT 189):

I. SEQUENCE CHARACTERISTICS:

(A) LENGTH: 20 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 72:

TTTTCATTTTGTTTTTTTTT

(74) INFORMATION FOR SEQ ID N°73 (IMT 179):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 73:

TTTTTTTTTTTTCATTTTGTTTTT

(75) INFORMATION FOR SEQ ID N°74 (IMT 191):

I. SEQUENCE CHARACTERISTICS:

(A) LENGTH: 28 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 74:

TTTTCATTTTGTTTTTTTTTTTTTTTTT

(76) INFORMATION FOR SEQ ID N°75 (IMT 608):

I. SEQUENCE CHARACTERISTICS:

(E) LENGTH: 24 base pairs

(F) TYPE: nucleic acid

(G) STRANDEDNESS: single

(H) TOPOLOGY: linear

II. MOLECULE TYPE: DNA

(A) SEQUENCE DESCRIPTION: SEQ ID N° 75:

TCATCAATTGGTCAATTGGTCATT

(77) INFORMATION FOR SEQ ID N°76 (IMT 609):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 76:

TCATCAAATGGTCAAATGGTCATT

(78) INFORMATION FOR SEQ ID N°77 (IMT 610):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 77:
TCATCAACTGGTCAACTGGTCATT

(79) INFORMATION FOR SEQ ID N°78 (IMT 611):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 78:
TCATCAAGTGGTCAAGTGGTCATT

(80) INFORMATION FOR SEQ ID N°79 (IMT 612):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 79:
TCATCAATAGGTCAATAGGTCATT

(81) INFORMATION FOR SEQ ID N°80 (IMT 613):
I. SEQUENCE CHARACTERISTICS:

(I) LENGTH: 24 base pairs
(A) TYPE: nucleic acid
(B) STRANDEDNESS: single
(C) TOPOLOGY: linear
II. MOLECULE TYPE: DNA
III. SEQUENCE DESCRIPTION: SEQ ID N° 80:
TCATCAATCGGTCAATCGGTCATT
(82) INFORMATION FOR SEQ ID N°81 (IMT 614):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 81:
TCATCAATGGGTCAATGGGTCATT
(83) INFORMATION FOR SEQ ID N°82 (IMT 539):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 82:
TCATCATTATGTCATTATGTCATT
(84) INFORMATION FOR SEQ ID N°83 (IMT 597):
I. SEQUENCE CHARACTERISTICS:
(A) LENGTH: 24 base pairs
(B) TYPE: nucleic acid
(C) STRANDEDNESS: single

(D) TOPOLOGY: linear
II. MOLECULE TYPE: DNA
III. SEQUENCE DESCRIPTION: SEQ ID N° 83:
TCATCATTCTGTCATTCTGTCATT

(85) INFORMATION FOR SEQ ID N°84 (IMT 602):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 84:
TCATCATTGTGTCATTGTGTCATT

(86) INFORMATION FOR SEQ ID N°85 (IMT 540):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 85:
TCATCATTTAGTCATTTAGTCATT

(87) INFORMATION FOR SEQ ID N°86 (IMT 598):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 86:

TCATCATTTCGTCATTTCGTCATT

(88) INFORMATION FOR SEQ ID N°87 (IMT 603):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 87:

TCATCATTTGGTCATTTGGTCATT

(89) INFORMATION FOR SEQ ID N°88 (IMT 544):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 88:

TCATCATTTTGACATTTTGACATT

(90) INFORMATION FOR SEQ ID N°89 (IMT 599):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 89:

TCATCATTTTGCCATTTTGCCATT

(91) INFORMATION FOR SEQ ID N°90 (IMT 604):

I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 90:
TCATCATTTTGGCATTTTGGCATT

(92) INFORMATION FOR SEQ ID N°91 ( IMT21 )
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 91:
TGCTGCTTTTGTGCTTTTGTGCTT

(93) INFORMATION FOR SEQ ID N°92 (IMT 595):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 92:
TCATCACTTTGGCACTTTGGCATT

(94) INFORMATION FOR SEQ ID N°93 (IMT 600):
I. SEQUENCE CHARACTERISTICS:
(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 93:

TCATCAGTTTGGCAGTTTGGCATT

(95) INFORMATION FOR SEQ ID N°94 (IMT 538):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 94:

TCATCATATTGGCATATTGGCATT

(96) INFORMATION FOR SEQ ID N°95 (IMT 596):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 95:

TCATCATCTTGGCATCTTGGCATT

(97) INFORMATION FOR SEQ ID N°96 (IMT 601):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 96:

TCATCATGTTGGCATGTTGGCATT

(98) INFORMATION FOR SEQ ID N°97 (IMT 023):

I. SEQUENCE CHARACTERISTICS:

(A) LENGTH: 24 base pairs

(B) TYPE: nucleic acid

(C) STRANDEDNESS: single

(D) TOPOLOGY' linear

II. MOLECULE TYPE. DNA

III. SEQUENCE DESCRIPTION: SEQ ID N° 97:

TGCTGCCCCCGCGCCCCCGCGCCC

(99) INFORMATION FOR SEQ ID N°98 (IMT 059):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 98

GGGGTTTTTTTTTTTTTTTTTTTT

(100) INFORMATION FOR SEQ ID N°99 (IMT 058):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 99:

GGGGTCTTTTTTTCTTTTTTTCTT

(101) INFORMATION FOR SEQ ID N°100 (IMT 037):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III SEQUENCE DESCRIPTION: SEQ ID N° 100:

TGCTGCCCCCGCGCCCCCGCGCCC

(102) INFORMATION FOR SEQ ID N°101 (IMT 034):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 101:

GGGGACTTTTATACTTTTATACTT

(103) INFORMATION FOR SEQ ID N°102 (IMT 057):

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N° 102:

GGGGTATTTTTTTATTTTTTTATT

(104) INFORMATION FOR SEQ ID N°103 (IMT 028):

I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 103:
GGGGGCTTTTGGGCTTTTGGGCGG

(105) INFORMATION FOR SEQ ID N°104 (IMT 040):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 104:
GGGGGCTTTAGTGCTTTAGTGCTT

(106) INFORMATION FOR SEQ ID N°105 (IMT 046):
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N° 105:
GGGGGCTAATGTGCTAATGTGCTT
(107) INFORMATION FOR SEQ ID N°106 (IMT 518):
I. SEQUENCE CHARACTERISTICS:
(A) LENGTH: 24 base pairs
(B) TYPE: nucleic acid

(C) STRANDEDNESS: single

(D) TOPOLOGY: linear

II. MOLECULE TYPE: DNA

III. SEQUENCE DESCRIPTION: SEQ ID N° 106:

TCGTCGAAAAGTCGAAAAGTCGAA

(108) INFORMATION FOR SEQ ID N°107 ( IMT105)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°107:

GGGGGTCTTTTTTTCTTTTTTTTT

(109) INFORMATION FOR SEQ ID N°108 ( IMT104)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°108:

TTTTTTCTTTTTTTCTTTTTTGGG

(110) INFORMATION FOR SEQ ID N°109 ( IMT106)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°109:

AAAAATCTTTTTTTCTTTTTTTTT

(111) INFORMATION FOR SEQ ID N°110 ( IMT166)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°110:

TGCTGCTTTTATGCTTTTATGCTT

(112) INFORMATION FOR SEQ ID N°111 ( IMT597)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°111:

TCATCATTCTGTCATTCTGTCATT

(113) INFORMATION FOR SEQ ID N°112 ( IMT101)

I SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°112:
TTTTTTCTTTTTTTCTTTTTTTTT

(114) INFORMATION FOR SEQ ID N°113 ( IMT169)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°113:
TGCTGCTTTTCTGCTTTTCTGCTT

(115) INFORMATION FOR SEQ ID N°114 ( IMT114)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°114:
TTTTTTCTTTTCTTTTTTTTTTTT

(116) INFORMATION FOR SEQ ID N°115 ( IMT111)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°115:

TTTTTCCTTTTTTCCTTTTTTTTT

(117) INFORMATION FOR SEQ ID N°116 ( IMT107)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°116:

CCCCCTCTTTTTTTCTTTTTTTTT

(118) INFORMATION FOR SEQ ID N°117 ( IMT113)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°117:

TTTTTTCTTTTTCTTTTTTTTTTT

(119) INFORMATION FOR SEQ ID N°118 ( IMT103)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°118:

TTTTTTCTTTTTTTCTTTTTTCCC

(120) INFORMATION FOR SEQ ID N°119 ( IMT186)

I. SEQUENCE CHARACTERISTICS:

(A) LENGTH: 24 base pairs

(B) TYPE: nucleic acid

(C) STRANDEDNESS: single

(D) TOPOLOGY. linear

II MOLECULE TYPE: DNA

III. SEQUENCE DESCRIPTION: SEQ ID N°119:

TTTTTTCTTTTTCTCTTTTTCTCT

(121) INFORMATION FOR SEQ ID N°120 ( IMT110)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°120:

TTTTTGCTTTTTTGCTTTTTTTTT

(122) INFORMATION FOR SEQ ID N°121 ( IMT109)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°121:

TTTTTACTTTTTTACTTTTTTTTT

(123) INFORMATION FOR SEQ ID N°122 ( IMT531)

I. SEQUENCE CHARACTERISTICS:

(A) LENGTH: 24 base pairs

(B) TYPE: nucleic acid

(C) STRANDEDNESS: single

(D) TOPOLOGY: linear

II MOLECULE TYPE: DNA

III. SEQUENCE DESCRIPTION SEQ ID N°122:

CATAATTTTGTAATTTTGTCATT

(124) INFORMATION FOR SEQ ID N°123 ( IMT102)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°123:

TTTTTTCTTTTTTTCTTTTTTAAA

(125) INFORMATION FOR SEQ ID N°124 ( IMT112)

I. SEQUENCE CHARACTERISTICS:

(A) LENGTH: 24 base pairs

(B) TYPE: nucleic acid

(C) STRANDEDNESS: single

(D) TOPOLOGY: linear

II. MOLECULE TYPE: DNA

III. SEQUENCE DESCRIPTION: SEQ ID N°124:

TTTTTTCTTTTTTTCTTTTTTTTT

(126) INFORMATION FOR SEQ ID N°125 (IMT 703)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°125:
TTTTTTTTTTTTCATTTTGTGGGG

(127) INFORMATION FOR SEQ ID N°126 (IMT 700)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°126:
TTTTTTTTTTTTCATTTTGTTTTG

(128) INFORMATION FOR SEQ ID N°127 (IMT 706)
I. SEQUENCE CHARACTERISTICS:
(A) LENGTH: 24 base pairs
(B) TYPE: nucleic acid
(C) STRANDEDNESS: single
(D) TOPOLOGY: linear
II. MOLECULE TYPE: DNA
III. SEQUENCE DESCRIPTION: SEQ ID N°127:
GGGTTTTTTTTTCATTTTGTTTTT

(129) INFORMATION FOR SEQ ID N°128 (IMT 712)

I. SEQUENCE CHARACTERISTICS:

(E) LENGTH: 24 base pairs

(F) TYPE: nucleic acid

(G) STRANDEDNESS: single

(H) TOPOLOGY: linear

II. MOLECULE TYPE: DNA

III SEQUENCE DESCRIPTION: SEQ ID Nº128:

GTTTTTTTTTTTCATTTTGTTTTG

## Claims

1. Use of an oligonucleotide having about 14 to 100 nucleotides for the manufacture of a medicament for inducing osteogenesis.

2. Use of an oligonucleotide having about 14 to 100 nucleotides for the manufacture of a medicament for the treatment of osteogenic dysfunctions.

3. Use according to claims 1 or 2 for the manufacture of a medicament for the treatment of osteoporosis.

4. Use according to claims 1 or 2 for the manufacture of a medicament for the treatment of skeletal deficiency or alteration.

5. Use according to anyone of the above claims **characterized in that** said oligonucleotide consists of 14 to 40 nucleotides.

6. Use according to anyone of the above claims **characterized in that** said oligonucleotide has any kind of modification of the natural (phosphodiester) phosphate backbone.

7. Use according to anyone of the above claims **characterized in that** said oligonucleotide has a phosphate backbone modification on the 5' internucleotide linkages.

8. Use according to anyone of the above claims **characterized in that** said oligonucleotide has a phosphate backbone modification on the 3' internucleotide linkages.

9. Use according to claims 7 or 8 **characterized in that** at least one of the internucleotide linkages is a phosphorotioate linkage.

10. Use according to anyone of the above claims **characterized in that** said oligonucleotide has at least one subsequence with the following composition: PyNTTTTNT, wherein Py is C or T and wherein N is any deoxyribonucleotide.

11. Use according to anyone of the above claims **characterized in that** said oligonucleotide has at least one subsequence with the following composition: PyNTTTTGT wherein Py is C or T and wherein N is any deoxyribonucleotide.

12. Use according to anyone of the above claims **characterized in that** said oligonucleotide is selected from the group consisting of:

TCATCATTTTGTCATTTTGTCATT (SEQ ID N°2);
TCCTCCTTTTGTCCTTTTGTCCTT (SEQ ID N°3);
TCTTCTTTTTGTCTTTTTGTCTTT (SEQ ID N°4);
TTGTTGTTTTGTTGTTTTGTTGTT (SEQ ID N°7);
TCATTTTTTTGTTTTTTTGTCATT (SEQ ID N°10);
TCATTGTTTTGTTGTTTTGTCATT (SEQ ID N°11);
TCATTCTTTTGTTCTTTTGTCATT (SEQ ID N°12);
TCATTATTTTGTTATTTTGTCATT (SEQ ID N°15);
TCATCCTTTTGTCCTTTTGTCATT (SEQ ID N°17);
TCATCTTTTTGTCTTTTTGTCATT (SEQ ID N°18);
CATTTTGTTTTTTTTTTTTTTTTT (SEQ ID N° 20);
TTCATTTTGTTTTTTTTTTTTTTT (SEQ ID N°21);
TTTTCATTTTGTTTTTTTTTTTTT (SEQ ID N°22);
TTTTTTCATTTTGTTTTTTTTTTT (SEQ ID N°23);
TTTTTTTTCATTTTGTTTTTTTTT (SEQ ID N°24);
TTTTTTTTTTCATTTTGTTTTTTT (SEQ ID N°25);
TTTTTTTTTTTTCATTTTGTTTTT (SEQ ID N°26);
TTTTTTTTTTTTTTCATTTTGTTT (SEQ ID N°27);
TTTTTTTTTTTTTTTTCATTTTGT (SEQ ID N° 28);
TTTTCATTTTGTCATTTTGTTTTT (SEQ ID N°29);
TCATCAATTTGTCAATTTGTCATT (SEQ ID N°30);
ACATCATTTTGTCATTTTGTCATT (SEQ ID N°46);
CCATCATTTTGTCATTTTGTCATT (SEQ ID N°47);
GCATCATTTTGTCATTTTGTCATT (SEQ ID N°48);
TAATCATTTTGTCATTTTGTCATT (SEQ ID N°49);
TTATCATTTTGTCATTTTGTCATT (SEQ ID N°50);
TGATCATTTTGTCATTTTGTCATT (SEQ ID N°51);
TCCTCATTTTGTCATTTTGTCATT (SEQ ID N°52);
TCTTCATTTTGTCATTTTGTCATT (SEQ ID N°53);
TCAACATTTTGTCATTTTGTCATT (SEQ ID N°55);
TCACCATTTTGTCATTTTGTCATT (SEQ ID N°56);
TCAGCATTTTGTCATTTTGTCATT (SEQ ID N°57);

TCATCATTTTGTCATTTTGTAATT (SEQ ID N°58);
TCATCATTTTGTCATTTTGTTATT (SEQ ID N°59);
TCATCATTTTGTCATTTTGTGATT (SEQ ID N°60);
TCATCATTTTGTCATTTTGTCCTT (SEQ ID N°61);
TCATCATTTTGTCATTTTGTCTTT (SEQ ID N°62);
TCATCATTTTGTCATTTTGTCAAT (SEQ ID N°64);
TCATCATTTTGTCATTTTGTCACT (SEQ ID N°65);
TCATCATTTTGTCATTTTGTCAGT (SEQ ID N°66);
TCATCATTTTGTCATTTTGTCATA (SEQ ID N°67);
TCATCATTTTGTCATTTTGTCATC (SEQ ID N°68);
TCATCATTTTGTCATTTTGTCATG (SEQ ID N°69);
TCATCAATTGGTCAATTGGTCATT (SEQ ID N°75);
TCATCAACTGGTCAACTGGTCATT (SEQ ID N°77);
TCATCATTGTGTCATTGTGTCATT (SEQ ID N°84);
TCATCATTTGGTCATTTGGTCATT (SEQ ID N°87);
TGCTGCTTTTGTGCTTTTGTGCTT (SEQ ID N°91);
TCATCATCTTGTCATCTTGTCATT (SEQ ID N°95);
TCATCATGTTGTCATGTTGTCATT (SEQ ID N°96);
GGGGGTCTTTTTTTCTTTTTTTTT (SEQ ID N°107)
TTTTTTCTTTTTTTCTTTTTTGGG (SEQ ID N°108)
AAAAATCTTTTTTTCTTTTTTTTT (SEQ ID N°109)
TGCTGCTTTTATGCTTTTATGCTT (SEQ ID N°110)
TCATCATTCTGTCATTCTGTCATT (SEQ ID N°111)
TTTTTTCTTTTTTTCTTTTTTTTT (SEQ ID N°112)
TGCTGCTTTTCTGCTTTTCTGCTT (SEQ ID N°113)
TTTTTTCTTTTCTTTTTTTTTTTT (SEQ ID N°114)
TTTTTCCTTTTTTCCTTTTTTTTT (SEQ ID N°115)
CCCCCTCTTTTTTTCTTTTTTTTT (SEQ ID N°116)
TTTTTTCTTTTTCTTTTTTTTTTT (SEQ ID N°117)
TTTTTTCTTTTTTTCTTTTTTCCC (SEQ ID N°118)
TTTTTTCTTTTTCTCTTTTTCTCT (SEQ ID N°119)
TTTTTGCTTTTTTGCTTTTTTTTT (SEQ ID N°120)

TTTTTACTTTTTTACTTTTTTTTT (SEQ ID N°121)
TCATAATTTTGTAATTTTGTCATT (SEQ ID N°122)
TTTTTTCTTTTTTTCTTTTTTAAA (SEQ ID N°123)
TTTTTTCTTTTTTCTTTTTTTTTT (SEQ ID N°124)
TTTTTTTTTTTTCATTTTGTGGGG (SEQ ID N°125)
TTTTTTTTTTTTCATTTTGTTTTG (SEQ ID N°126)
GGGTTTTTTTTTCATTTTGTTTTT (SEQ ID N°127)
GTTTTTTTTTTTCATTTTGTTTTG (SEQ ID N°128)

13. Use according to anyone of the above claims **characterized in that** said medicament is administered to a human.

14. Use according to anyone of the above claims **characterized in that** said oligonucleotide is encapsulated in a slow release delivery vehicle.

15. Use according to anyone of the above claims **characterized in that** said oligonucleotide is included in a pharmaceutically acceptable carrier.

16. Use according to anyone of the above claims **characterized in that** said oligonucleotide is encapsulated in a slow release delivery vehicle.

17. Use according to anyone of the above claims **characterized in that** said oligonucleotide osteogenic oligonucleotide is administered to a subject combined with a device aimed to aid in the treatment of a skeletal deficiency or alteration.

18. Use according to claim 17 **characterized in that** said device is a molded implant, a prosthetic device, a capsule, titanium alloy, or a ceramic block.

19. Use according to claim 18 **characterized in that** said device is a ceramic block comprising 0-100 % hydroxylapatite and the remaining 100-0% tricalcium phosphate, by weight.

20. Use according to claim 18 **characterized in that** said medicament is combined with the device by adsorption, impregnation or inclusion into holes or channels performed in the device to hold the preparation.

21. Use according to claim 20 with the addition of an osteogenic cell source.

22. Use according to anyone of the above claims **characterized in that** said oligonucleotide is present in amounts of 1 µg to 100mg per dose.

23. Use according to anyone of the above claims **characterized in that** said medicament is selected from the group consisting of liquid, gel and lyophilized formulations.

24. Use according to anyone of the above claims **characterized in that** said medicament is administered by intradermic, intramuscular or intravenous injection.

25. Use according to anyone of the above claims **characterized in that** said medicament is administered by an oral, intranasal, anal, vaginal, or transdermal route.
